# EUROPEAN PATENT APPLICATION

(11) **EP 4 170 027 A1**
(43) Date of publication of application: **26.04.2023**
(21) Application number: 21827034.6
(22) Date of filing: 06.04.2021
(51) Int. Cl.: C12N 15/09, C12M 1/34, C12Q 1/68, G01N 33/53, G16B 40/20

(54) **INFORMATION PROCESSING DEVICE, OPERATION METHOD FOR INFORMATION PROCESSING DEVICE, AND OPERATION PROGRAM FOR INFORMATION PROCESSING DEVICE**

(30) Priority: 19.06.2020 JP 2020106417
(71) Applicant: FUJIFILM Corporation, Tokyo 106-8620 (JP)
(72) Inventor: NAGASE, Masaya, Ashigarakami-gun, Kanagawa 258-8577 (JP)
(74) Representative: Klunker IP Patentanwälte PartG mbB
(86) International application number: PCT/JP2021/014592
(87) International publication number: WO 2021/256055

(57) **Abstract**

There is provided an operation method for an information processing apparatus, the operation method being performed by a processor, the operation method including: an acquisition process for acquiring pieces of annotation information added to each of a plurality of biomarkers related to biological samples; a deriving process for deriving an evaluation value of each of the plurality of biomarkers on the basis of the pieces of annotation information; and a selection process for selecting on the basis of the evaluation value, measurement target biomarkers from among the plurality of biomarkers.

## Description

### BACKGROUND OF THE INVENTION

### 1. Field of the Invention

The present disclosure relates to a technique for an information processing apparatus, an operation method for an information processing apparatus, and an operation program for an information processing apparatus.

### 2. Description of the Related Art

In a field of study that focuses on a biological sample of, for example, iPS cells (induced Pluripotent Stem cells) as a study subject, a multilevel experiment for which parameters including variations of cell clones and the dosages of drugs are varied is planned, and with reference to biomarkers acquired as a result of the experiment, the characteristics including the differentiation potency of the biological sample is elucidated. The biomarkers include, for example, genes and protein expressed by the cells during culture, metabolites produced from the cells during culture, or elements related to the environment for culturing the cells, such as the carbon dioxide concentration and pH (potential of Hydrogen).

As a test of genes, which are a typical example of the biomarkers, RNA (Ribonucleic Acid) sequencing (RNA-Seq) is known. RNA-Seq enables comprehensive measurement of the expression levels of tens of thousands of genes. This expedites elucidation of the characteristics of the biological sample. However, this test takes time and incurs relatively high costs, and therefore, has difficulty in deployment to multilevel experiments.

Genes exist in enormous numbers, and some genes are less likely to contribute to elucidation of the characteristics of the biological sample. Accordingly, to elucidate the characteristics of the biological sample more effectively, it is important to narrow down an enormous number of genes and select genes that are considered to contribute to elucidation of the characteristics of the biological sample as measurement targets for multilevel experiments.

As existing methods for selecting genes that are measurement targets, the following two methods are available. A first method is a method based on researchers' experience and knowledge. Specifically, previously known genes that are genes already known to influence cell behaviors are selected as measurement targets. A second method is a method of selecting genes on the basis of the actual measurement results of the expression levels of genes in a data-driven manner. Specifically, a preliminary experiment is carried out with a sample in small quantity and the expression levels of genes are comprehensively measured, and thereafter, some of the differentially expressed genes (DEGs), which are genes having significantly different expression levels, are selected as measurement targets. For example, <Aravind Subramanian et al. "A Next Generation Connectivity Map: L1000 Platform and the First 1,000,000 profiles", published on November 30, 2015, Cell, Volume 171, ISSUE 6, Pages 1437-1452.e17, Internet <URL:https://www.ncbi.nlm.nih.gov/pmc/articles/PMC5990023/>> describes a method of selecting, from among DEGs extracted in a data-driven manner, DEGs with which 80% or more of the behaviors of all genes can be explained are selected as measurement targets with an advanced data analysis technique.

### SUMMARY OF THE INVENTION

However, the first method of selecting previously known genes as measurement targets depends on researchers' experience and knowledge, which imposes a limitation on the number of previously known genes, and therefore, might not be able to appropriately select genes that are considered to contribute to elucidation of the characteristics of the biological sample. In the second method of selecting some DEGs as measurement targets, genes are simply selected because the amount of difference is significant, and therefore, when minor genes for which researchers have little knowledge are found to specifically contribute to elucidation of the characteristics of the biological sample as a result of deployment to a multilevel experiment, it may be difficult to develop a guideline about how to improve the cell culturing performance.

One embodiment of the technique of the present disclosure provides an information processing apparatus, an operation method for an information processing apparatus, and an operation program for an information processing apparatus that allow selection of more appropriate measurement target biomarkers leading to elucidation of the characteristics of the biological sample.

An operation method for an information processing apparatus according to the present disclosure is performed by a processor, the operation method including: an acquisition process for acquiring pieces of annotation information added to each of a plurality of biomarkers related to biological samples; a deriving process for deriving an evaluation value of each of the plurality of biomarkers on the basis of the pieces of annotation information; and a selection process for selecting on the basis of the evaluation value, measurement target biomarkers from among the plurality of biomarkers.

Preferably, the processor is configured to select pieces of annotation information related to a biological-sample's characteristic of interest, and derive the evaluation value on the basis of only the selected pieces of annotation information.

Preferably, the processor is configured to add the pieces of annotation information to the biomarkers with reference to a database in which the pieces of annotation information for the biomarkers are registered.

Preferably, the pieces of annotation information are associated with types of the biological samples.

Preferably, the processor is configured to accept a plurality of categories defined in accordance with the types of the biological samples and a range of the number of measurement target biomarkers for each of the plurality of categories, the plurality of categories and the range being specified by a user, and select a number of biomarkers, the number satisfying the range, from among biomarkers prepared for each category of the plurality of categories, and sort the selected biomarkers into the category as the measurement target biomarkers.

Preferably, the categories include categories of iPS cell, ectoderm, mesoderm, and endoderm.

Preferably, the processor is configured to count an addition number that is the number of pieces of annotation information added to each of the plurality of biomarkers, and derive the evaluation value on the basis of the addition number.

Preferably, the processor is configured to assign a weight to the evaluation value in accordance with information values of the pieces of annotation information.

Preferably, the processor is configured to determine a piece of annotation information having a relatively high rarity to have a high information value and increase a weight of the piece of annotation information.

Preferably, the processor is configured to assign the weight to the evaluation value on the basis of orthogonality of the pieces of annotation information.

Preferably, the processor is configured to increase a weight of an evaluation value of a biomarker having a strength indicator that is within a preset threshold range.

Preferably, the processor is configured to accept previously known markers specified by a user, the previously known markers being biomarkers already known to influence a characteristic of the biological samples, and increase weights of evaluation values of the previously known markers.

Preferably, the processor is configured to select as the measurement target biomarkers, more than 100 and less than or equal to 1000 biomarkers.

Preferably, the biomarkers include genes.

Preferably, the genes include differentially expressed genes having significantly different expression levels.

Preferably, each of the pieces of annotation information includes a term defined by Gene Ontology.

Preferably, the processor is configured to acquire measurement results regarding the measurement target biomarkers, pick, on the basis of the measurement results, pieces of annotation information that influence a characteristic of the biological samples to a relatively large degree from among pieces of annotation information added to the measurement target biomarkers, with a statistical method, and present the picked pieces of annotation information to a user.

An information processing apparatus according to the present disclosure includes at least one processor, the processor being configured to acquire pieces of annotation information added to each of a plurality of biomarkers related to biological samples, derive an evaluation value of each of the plurality of biomarkers on the basis of the pieces of annotation information, and select on the basis of the evaluation value, measurement target biomarkers from among the plurality of biomarkers.

An operation program for an information processing apparatus according to the present disclosure is for causing a processor to perform an acquisition process for acquiring pieces of annotation information added to each of a plurality of biomarkers related to biological samples; a deriving process for deriving an evaluation value of each of the plurality of biomarkers on the basis of the pieces of annotation information; and a selection process for selecting on the basis of the evaluation value, measurement target biomarkers from among the plurality of biomarkers.

With the technique of the present disclosure, it is possible to provide an information processing apparatus, an operation method for an information processing apparatus, and an operation program for an information processing apparatus that allow selection of more appropriate measurement target biomarkers leading to elucidation of the characteristics of the biological sample.

### BRIEF DESCRIPTION OF THE DRAWINGS

Fig. 1 is a diagram illustrating an information processing apparatus and so on;
Fig. 2 is a diagram illustrating gene expression information;
Fig. 3 is a diagram illustrating an annotation information table;
Fig. 4 is a table showing pieces of annotation information;
Fig. 5 is a diagram illustrating a state where an iPS cell differentiates into three germ layers and the three germ layers differentiate into tissue cells;
Fig. 6 is a diagram illustrating an overview of processing by the information processing apparatus;
Fig. 7 is a block diagram of a computer that forms the information processing apparatus;
Fig. 8 is a block diagram of processing units in a CPU of the information processing apparatus;
Fig. 9 is a diagram illustrating a category specifying screen and category and number range specifying information;
Fig. 10 is a diagram illustrating a state where a warning screen is displayed on the category specifying screen as a pop-up screen;
Fig. 11 is a diagram illustrating an overview of processing by a selection unit;
Fig. 12 is a diagram illustrating a previously known gene specifying screen and previously known gene specifying information;
Fig. 13 is a diagram illustrating an extraction target specifying screen and extraction target specifying information;
Fig. 14 is a diagram illustrating a DEGs list;
Fig. 15 is a diagram illustrating delivery information;
Fig. 16 is a diagram illustrating a state where an acquisition unit generates a DEGs-with-addition list;
Fig. 17 is a diagram illustrating a state where a deriving unit generates an evaluation value table;
Fig. 18 is a diagram illustrating a state where the selection unit unconditionally selects previously known genes as measurement target genes;
Fig. 19 is a diagram illustrating a state where the selection unit generates a selection priority table group from the evaluation value table;
Fig. 20 is a diagram illustrating a state where the selection unit selects a number of DEGs, the number satisfying a number range, and sorts the selected DEGs as measurement target genes;
Fig. 21 is a diagram illustrating a measurement target gene list;
Fig. 22 is a diagram illustrating an overview of processing by an extraction unit and by the acquisition unit;
Fig. 23 is a diagram illustrating an overview of processing by the deriving unit and by the selection unit;
Fig. 24 is a diagram illustrating a measurement target gene display screen;
Fig. 25 is a flowchart illustrating a processing procedure in the information processing apparatus;
Fig. 26 is a diagram illustrating an example where a piece of annotation information having a relatively high rarity is determined to have a high information value and the addition number of the piece of annotation information is increased;
Fig. 27 is a table showing the state of addition of pieces of annotation information to three DEGs;
Fig. 28 is a diagram illustrating a third embodiment in which the weights of the evaluation values of genes each having a strength indicator that is within a preset threshold range are increased;
Fig. 29 is a diagram illustrating a fourth embodiment in which the measurement results of the expression levels of measurement target genes are acquired and pieces of highly influential annotation information are picked, on the basis of the measurement results;
Fig. 30 is a flowchart illustrating a processing procedure for picking pieces of highly influential annotation information by a picking unit;
Fig. 31 is a diagram illustrating a state where the picking unit extracts highly expressed genes from among measurement target genes with reference to measurement results;
Fig. 32 is a diagram illustrating a state where the picking unit extracts pieces of annotation information added to highly expressed genes from a DEGs-with-addition list;
Fig. 33 is a diagram illustrating a state where the picking unit calculates an odds ratio and a p-value for each of the pieces of annotation information added to highly expressed genes and picks pieces of annotation information each having a p-value that is less than 0.05 as pieces of highly influential annotation information;
Fig. 34 is a diagram illustrating a highly influential annotation information display screen;
Fig. 35 is a table showing previously known genes specified in order to select C1000 that includes measurement target genes in an example and extracted DEGs;
Fig. 36 is a diagram illustrating the measurement results of the expression levels of a microarray in a comparative example;
Fig. 37 is a table showing pieces of highly influential annotation information picked on the basis of genes used in measurement with the microarray;
Fig. 38 is a table showing pieces of highly influential annotation information picked on the basis of genes used in measurement with the microarray;
Fig. 39 is a diagram illustrating the measurement results of the expression levels of C 1000;
Fig. 40 is a table showing pieces of highly influential annotation information picked on the basis of C1000 and genes to which the pieces of highly influential annotation information are added;
Fig. 41 is a bar chart of odds ratios based on the set of measurement genes in C1000; and
Fig. 42 is a bar chart of odds ratios based on a set of measurement genes of TaqMan Scorecard in the comparative example.

### DESCRIPTION OF THE PREFERRED EMBODIMENTS

### First Embodiment

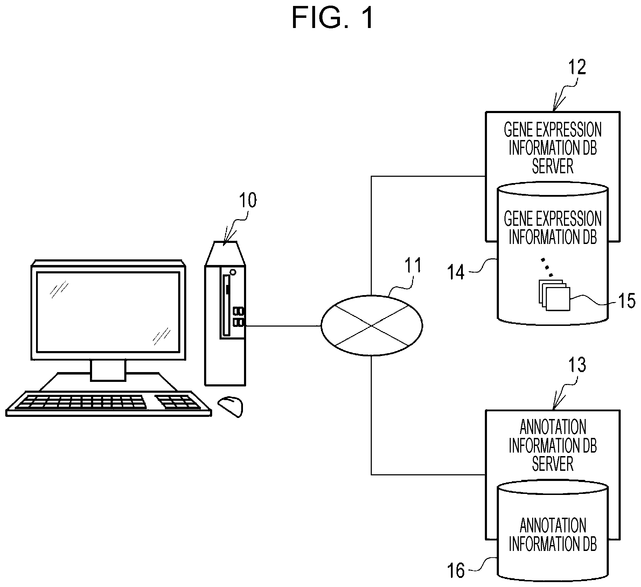

Fig. 1 illustrates an information processing apparatus 10 that is, for example, a desktop personal computer and that is operated by a user who is, for example, a researcher studying cells, which are an example of "biological sample" in the technique of the present disclosure. The information processing apparatus 10 is connected to a network 11. The network 11 is, for example, the Internet or a WAN (wide area network), such as a public communication network.

The information processing apparatus 10 is connected to a gene expression information database (hereinafter abbreviated as "DB") server 12 and to an annotation information DB server 13 over the network 11. The gene expression information DB server 12 has a gene expression information DB 14. The gene expression information DB 14 is, for example, GEO (Gene Expression Omnibus) provided by National Center for Biotechnology Information (NCBI). In the gene expression information DB 14, an enormous number of pieces of gene expression information 15 uploaded from an indefinite number of researchers are registered as open data. Each of the pieces of gene expression information 15 is information regarding the levels of genes expressed by cells during culture, that is, information regarding the expression levels. Genes are an example of "biomarkers" in the technique of the present disclosure.

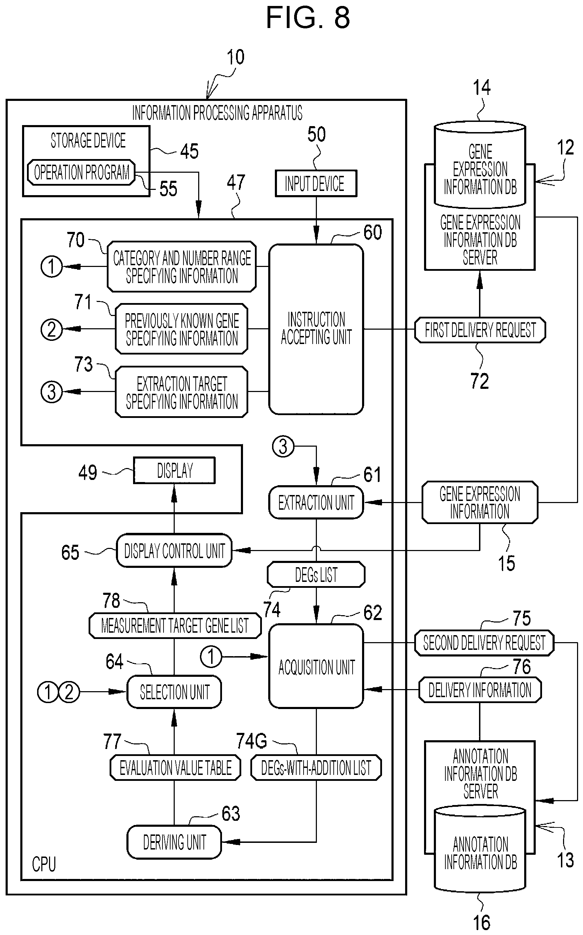

The gene expression information DB server 12 receives a first delivery request 72 (see Fig. 8) from the information processing apparatus 10. The gene expression information DB server 12 reads from the gene expression information DB 14, pieces of gene expression information 15 corresponding to the first delivery request 72. The gene expression information DB server 12 delivers the read pieces of gene expression information 15 to the information processing apparatus 10.

The annotation information DB server 13 has an annotation information DB 16. The annotation information DB 16 is, for example, DAVID (The Database for Annotation, Visualization and Integrated Discovery) provided by National Institute of Allergy and Infectious Diseases (NIAID) and/or InterPro provided by European Bioinformatics Institute (EBI). In the annotation information DB 16, for each of a plurality of genes, corresponding pieces of annotation information are registered. That is, the annotation information DB 16 is an example of "database" in the technique of the present disclosure.

The annotation information DB server 13 receives a second delivery request 75 (see Fig. 8) from the information processing apparatus 10. The annotation information DB server 13 reads from the annotation information DB 16, pieces of annotation information corresponding to the second delivery request 75. The annotation information DB server 13 delivers delivery information 76 (see Fig. 8) including the read pieces of annotation information to the information processing apparatus 10.

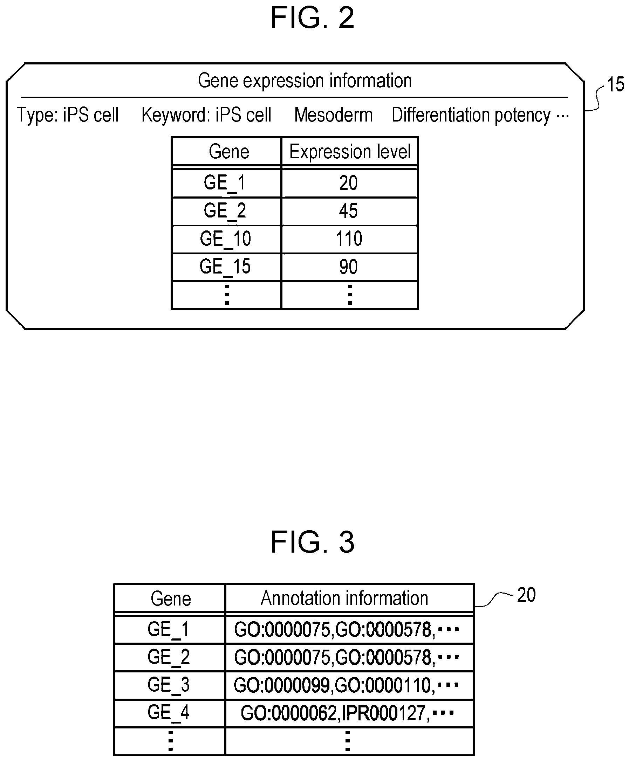

As illustrated in Fig. 2, the gene expression information 15 is information in which the expression levels of respective genes are registered. In the gene expression information 15, the type of a biological sample (in Fig. 2, "iPS cell") for which the expression levels are measured is registered. In the gene expression information 15, keywords including "iPS cell", "mesoderm", "differentiation potency", and so on for facilitating searches are registered. Keywords are registered by, for example, a researcher who has uploaded the gene expression information 15 or the provider of the gene expression information DB 14.

In the annotation information DB 16, an annotation information table 20 illustrated in Fig. 3 is stored. In the annotation information table 20, IDs (pieces of identification data) of pieces of annotation information are registered for each of the genes.

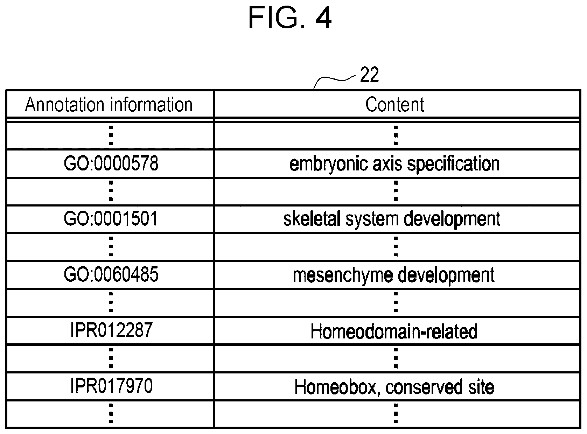

As shown by a table 22 in Fig. 4, annotation information includes a term defined by Gene Ontology (GO), such as "embryonic axis specification" for ID "G0:0000578" or "Homeodomain-related" for ID "IPR012287".

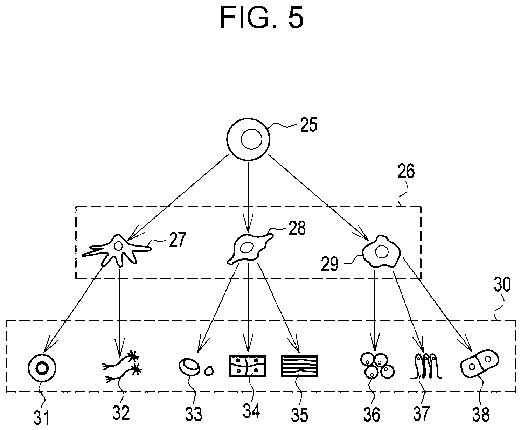

An example case where an iPS cell 25 illustrated in Fig. 5 that is established by initializing a human somatic cell is a study subject will be described below. The iPS cell 25 undergoes a cell division and forms three germ layers 26. The three germ layers 26 include an ectoderm 27, a mesoderm 28, and an endoderm 29. The three germ layers 26 each differentiates into a plurality of types of tissue cells 30. Specifically, the ectoderm 27 differentiates into a crystalline 31, a nerve cell 32, and so on. The mesoderm 28 differentiates into a blood cell 33, a bone cell 34, a muscle cell 35, and so on. The endoderm 29 differentiates into an alveolar cell 36, an intestinal cell 37, a liver cell 38, and so on.

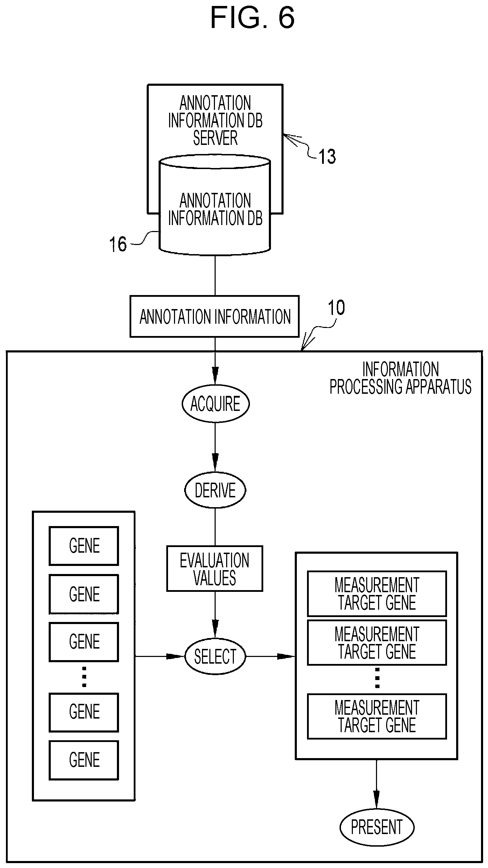

Fig. 6 illustrates an overview of processing by the information processing apparatus 10. The information processing apparatus 10 first acquires pieces of annotation information from the annotation information DB server 13. The information processing apparatus 10 derives the evaluation values of respective genes on the basis of the acquired pieces of annotation information. Next, the information processing apparatus 10 selects on the basis of the derived evaluation values, genes that are measurement targets (hereinafter referred to as "measurement target genes") from among the plurality of genes. At this time, the information processing apparatus 10 selects a user-specified number of measurement target genes. The number of genes that are potential measurement target genes is, for example, about 3000 and the number of measurement target genes is, for example, 1000. The information processing apparatus 10 presents the selected measurement target genes to the user. The measurement target genes are an example of "measurement target biomarkers" in the technique of the present disclosure.

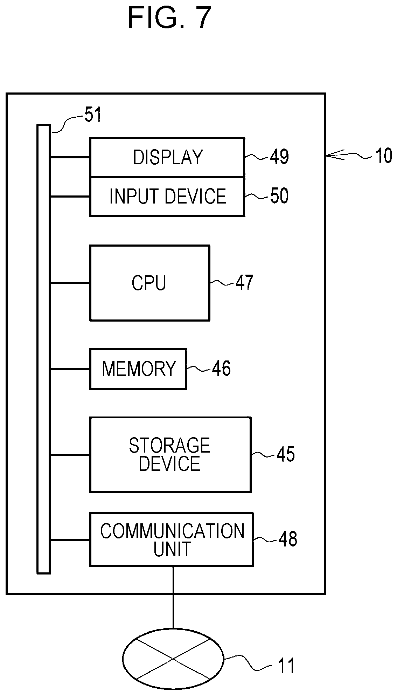

Fig. 7 illustrates the computer that forms the information processing apparatus 10 and that includes a storage device 45, a memory 46, a CPU (central processing unit) 47, a communication unit 48, a display 49, and an input device 50. These are connected to each other via a bus line 51.

The storage device 45 is a hard disk drive that is built in the computer that forms the information processing apparatus 10 or that is connected to the information processing apparatus 10 by a cable or over a network. Alternatively, the storage device 45 is a disk array formed of a plurality of hard disk drives. In the storage device 45, a control program, such as an operating system, various application programs, various types of data associated with these programs, and so on are stored. Instead of the hard disk drive, a solid state drive may be used.

The memory 46 is a work memory for the CPU 47 to perform processing. The CPU 47 loads a program stored in the storage device 45 to the memory 46 and performs processing in accordance with the program. Accordingly, the CPU 47 centrally controls the units of the computer.

The communication unit 48 is a network interface for controlling transmission of various types of information over the network 11. The display 49 displays various screens. The computer that forms the information processing apparatus 10 accepts, on various screens, operation instructions input by using the input device 50. The input device 50 includes a keyboard, a mouse, a touch panel, and so on.

Fig. 8 illustrates the storage device 45 of the information processing apparatus 10 in which an operation program 55 is stored. The operation program 55 is an application program for causing the computer to function as the information processing apparatus 10. That is, the operation program 55 is an example of "an operation program for an information processing apparatus" in the technique of the present disclosure.

In response to the start of the operation program 55, the CPU 47 of the computer that forms the information processing apparatus 10 cooperates with the memory 46 and so on to function as an instruction accepting unit 60, an extraction unit 61, an acquisition unit 62, a deriving unit 63, a selection unit 64, and a display control unit 65. The CPU 47 is an example of "processor" in the technique of the present disclosure.

The instruction accepting unit 60 accepts various instructions given by the user using the input device 50. For example, the instruction accepting unit 60 accepts a plurality of categories and the range of the number of measurement target genes (hereinafter referred to as "number range") for each of the plurality of categories, the plurality of categories and the number ranges being specified by the user. The categories are defined by the user in accordance with the types of biological samples. The instruction accepting unit 60 generates category and number range specifying information 70 corresponding to the specified categories and the specified number ranges and outputs the category and number range specifying information 70 to the selection unit 64.

The instruction accepting unit 60 also accepts previously known genes specified by the user. The instruction accepting unit 60 generates previously known gene specifying information 71 corresponding to the specified previously known genes and outputs the previously known gene specifying information 71 to the selection unit 64. The previously known genes are genes that are already known to influence a behavior of the iPS cell 25. That is, the previously known genes are an example of "previously known markers" in the technique of the present disclosure. The behavior of the iPS cell 25 is an example of "a characteristic of the biological samples" in the technique of the present disclosure.

The instruction accepting unit 60 also accepts a first delivery instruction given by the user for instructing the gene expression information DB server 12 to deliver pieces of gene expression information 15. The first delivery instruction is specifically a search instruction including search keywords related to the iPS cell 25, such as "iPS cell", "ectoderm", "endoderm", "mesoderm", and so on. The first delivery instruction is given on a search screen (not illustrated) on which input boxes for search keywords and a search button are provided. When accepting the first delivery instruction, the instruction accepting unit 60 transmits the first delivery request 72 including the above-described search keywords to the gene expression information DB server 12. The gene expression information DB server 12 retrieves pieces of gene expression information 15 including registered keywords that match the search keywords among pieces of gene expression information 15 stored in the gene expression information DB 14. The gene expression information DB server 12 delivers the retrieved pieces of gene expression information 15 to the information processing apparatus 10. The pieces of gene expression information 15 are input to the extraction unit 61 and the display control unit 65 in the information processing apparatus 10.

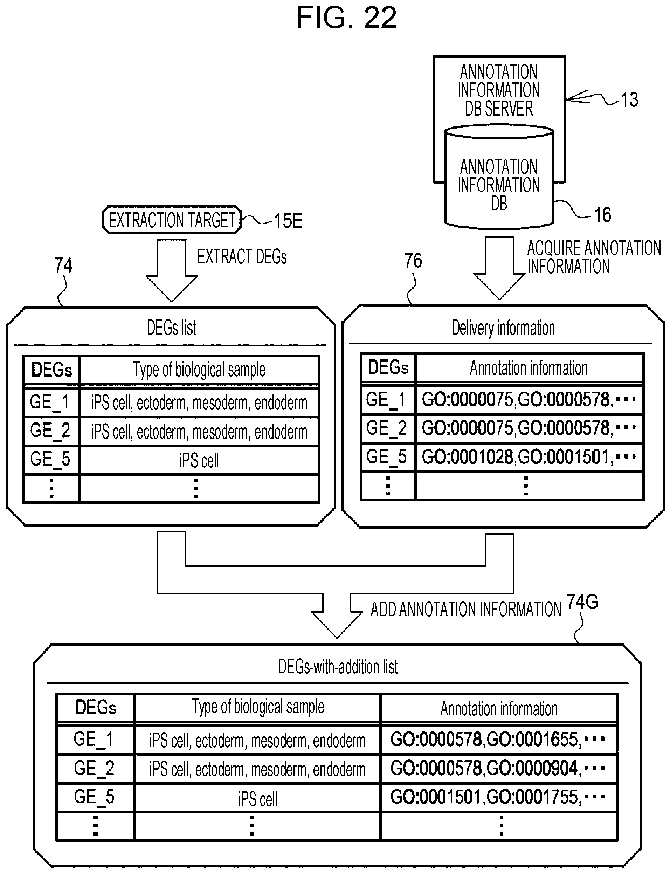

The display control unit 65 displays on the display 49 a display screen (not illustrated) for the pieces of gene expression information 15 from the gene expression information DB server 12. The instruction accepting unit 60 accepts pieces of gene expression information 15 that are DEGs extraction targets (hereinafter referred to as "extraction targets 15E" (see Fig. 22)) specified by the user from among the displayed pieces of gene expression information 15. The instruction accepting unit 60 generates extraction target specifying information 73 corresponding to the specified extraction targets 15E and outputs the extraction target specifying information 73 to the extraction unit 61.

The extraction unit 61 extracts DEGs from the extraction targets 15E specified in the extraction target specifying information 73. The extraction unit 61, for example, compares the expression level of each of the genes in each of the extraction targets 15E with a preset threshold value and extracts genes each having an expression level that is greater than or equal to the threshold value as DEGs. The extraction unit 61 generates a DEGs list 74 in which the extracted DEGs are registered and outputs the DEGs list 74 to the acquisition unit 62.

The acquisition unit 62 transmits to the annotation information DB server 13 the second delivery request 75 based on the DEGs list 74 from the extraction unit 61. The second delivery request 75 includes the DEGs registered in the DEGs list 74. The annotation information DB server 13 retrieves pieces of annotation information added to the DEGs included in the second delivery request 75, from the annotation information table 20 in the annotation information DB 16. The annotation information DB server 13 delivers the delivery information 76 including sets of the retrieved pieces of annotation information and the DEGs to the information processing apparatus 10. The delivery information 76 is input to the acquisition unit 62 in the information processing apparatus 10.

The acquisition unit 62 acquires the delivery information 76 from the annotation information DB server 13. The delivery information 76 includes the pieces of annotation information as described above. Accordingly, the acquisition unit 62 acquires the delivery information 76 to thereby consequently acquire the pieces of annotation information.

The acquisition unit 62 adds the pieces annotation information to the DEGs list 74 on the basis of the delivery information 76 to thereby create a DEGs-with-addition list 74G from the DEGs list 74. That is, the acquisition unit 62 adds the pieces of annotation information to the genes with reference to the annotation information DB 16. The acquisition unit 62 outputs the DEGs-with-addition list 74G to the deriving unit 63.

The deriving unit 63 derives the evaluation values of the respective DEGs on the basis of the DEGs-with-addition list 74G. The deriving unit 63 outputs an evaluation value table 77 that includes the results of deriving the evaluation values to the selection unit 64.

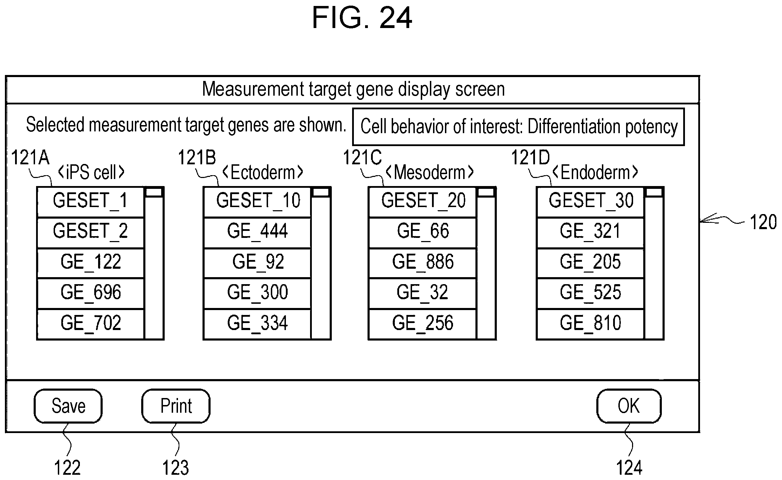

The selection unit 64 unconditionally selects the previously known genes in accordance with the previously known gene specifying information 71 as measurement target genes. The selection unit 64 selects in accordance with the category and number range specifying information 70, measurement target genes from among the DEGs extracted by the extraction unit 61. The selection unit 64 outputs a measurement target gene list 78 that includes the results of selecting the measurement target genes to the display control unit 65. The display control unit 65 generates and displays on the display 49 a measurement target gene display screen 120 (see Fig. 24) on the basis of the measurement target gene list 78.

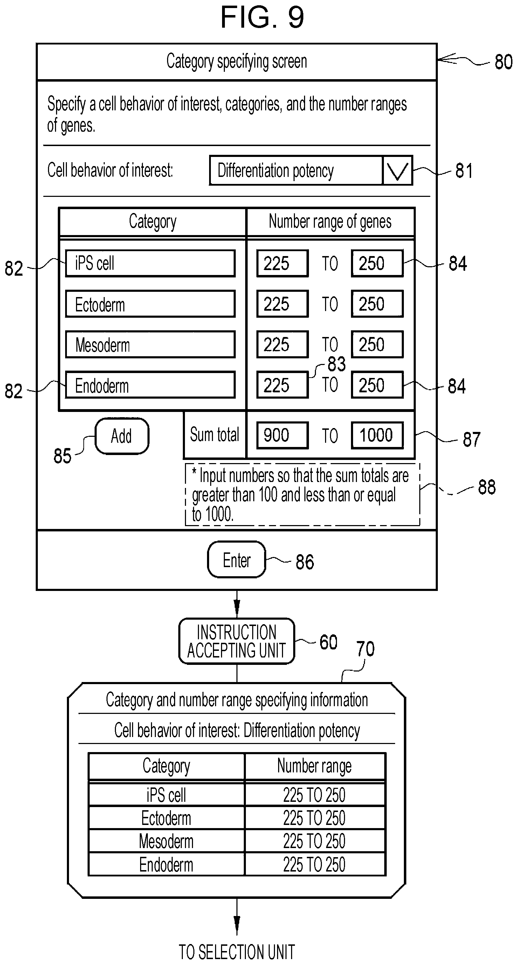

Fig. 9 illustrates a category specifying screen 80 that is displayed on the display 49 under the control of the display control unit 65 for accepting categories and number ranges specified by the user. On the category specifying screen 80, a pull-down menu 81 for selecting and inputting a cell behavior of interest, which is an example of "biological-sample's characteristic of interest" in the technique of the present disclosure, is provided. On the category specifying screen 80, input boxes 82 for categories, input boxes 83 for the lower limits of number ranges, and input boxes 84 for the upper limits of the number ranges are provided. Additional input boxes 82 to 84 can be added by selecting an add button 85.

When a cell behavior of interest is selected from the pull-down menu 81, desired categories and number ranges are input to the input boxes 82 to 84, and an enter button 86 is selected, the instruction accepting unit 60 accepts the specified cell behavior of interest, categories, and number ranges. Accordingly, the category and number range specifying information 70 is output to the selection unit 64 from the instruction accepting unit 60. The category and number range specifying information 70 includes the cell behavior of interest selected from the pull-down menu 81, the categories input to the input boxes 82, and the number ranges input to the input boxes 83 and 84.

Fig. 9 illustrates an example case where "differentiation potency" is selected as the cell behavior of interest. Fig. 9 illustrates an example case where "iPS cell", "ectoderm", "mesoderm", and "endoderm" are specified as the categories and "225 to 250" is specified for each of the categories as the number range. Note that one category may be specified. Further, the same numerical values may be input to the input boxes 83 and 84.

Below the input boxes 83 and 84, a display region 87 for displaying the sum totals of the lower limits and the upper limits of the number ranges input to the input boxes 83 and 84 is provided. Below the display region 87, a message 88 for prompting the user to limit the sum totals so as to be greater than 100 and less than or equal to 1000 is displayed.

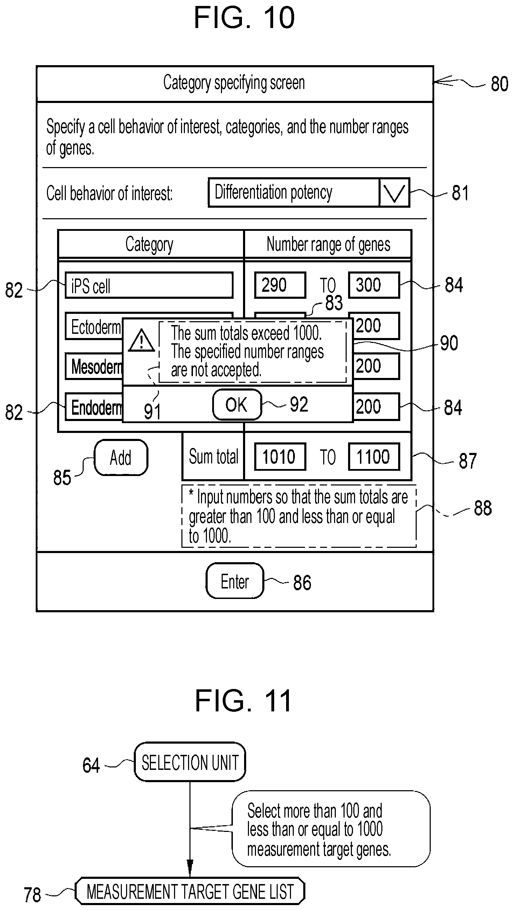

When the enter button 86 is selected while the sum totals are outside a range of greater than 100 and less than or equal to 1000, the display control unit 65 displays a warning screen 90 on the category specifying screen 80 as a pop-up screen as illustrated in Fig. 10. On the warning screen 90, a message 91 that the sum totals are outside a range of greater than 100 and less than or equal to 1000 and the specified number ranges are not accepted as is, is displayed. When on OK button 92 is selected, the display control unit 65 hides the warning screen 90.

The category specifying screen 80 is thus configured so as not to accept the specified number ranges when the sum totals are outside a range of greater than 100 and less than or equal to 1000. Accordingly, the selection unit 64 consequently selects more than 100 and less than or equal to 1000 measurement target genes as illustrated in Fig. 11.

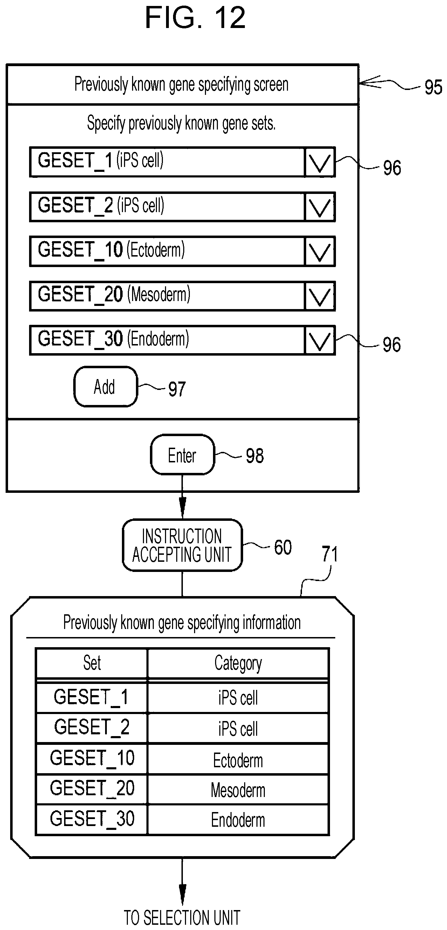

Fig. 12 illustrates a previously known gene specifying screen 95 that is displayed on the display 49 under the control of the display control unit 65 for accepting previously known genes specified by the user. On the previously known gene specifying screen 95, pull-down menus 96 for selecting and inputting previously known gene sets are provided. An additional pull-down menu 96 can be added by selecting an add button 97. Each of the pull-down menus 96 includes a plurality of previously known gene sets prepared in advance as choices. The previously known gene sets are prepared for each of the categories. Examples of the previously known gene sets include previously known gene sets used in a gene analysis using TaqMan (registered trademark) Scorecard, previously known gene sets used in a gene analysis using nCounter (registered trademark), and previously known gene sets used in a gene analysis using TruSeq (registered trademark).

When desired previously known gene sets are selected from the pull-down menus 96 and an enter button 98 is selected, the instruction accepting unit 60 accepts the specified previously known gene sets. Accordingly, the previously known gene specifying information 71 is output to the selection unit 64 from the instruction accepting unit 60. The previously known gene specifying information 71 is information in which the previously known gene sets and categories corresponding to the respective previously known gene sets are registered.

Fig. 12 illustrates an example case where two previously known gene sets are specified for the category "iPS cell" and one previously known gene set is specified for each of the categories "ectoderm" "mesoderm", and "endoderm", that is, five previously known gene sets are specified in total. Note that instead of or in addition to specifying sets, previously known genes may be specified one by one.

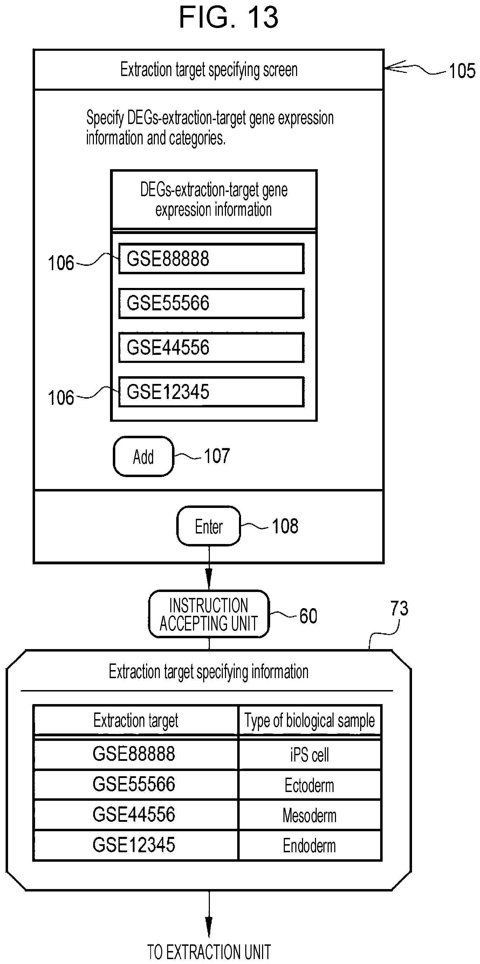

Fig. 13 illustrates an extraction target specifying screen 105 that is displayed on the display 49 under the control of the display control unit 65 for allowing the user to specify the extraction targets 15E from among the pieces of gene expression information 15 from the gene expression information DB server 12. On the extraction target specifying screen 105, input boxes 106 for the extraction targets 15E are provided. An additional input box 106 can be added by selecting an add button 107.

When the extraction targets 15E are input to the input boxes 106 and an enter button 108 are selected, the instruction accepting unit 60 accepts the specified extraction targets 15E. Accordingly, the extraction target specifying information 73 is output to the extraction unit 61 from the instruction accepting unit 60. The extraction target specifying information 73 is information in which the extraction targets 15E input to the input boxes 106 and the types of biological samples registered for the respective extraction targets 15E are registered.

Fig. 13 illustrates an example case where one extraction target 15E is specified for each of the types of biological samples, namely, "iPS cell", "ectoderm", "mesoderm", and "endoderm". Note that two or more extraction targets 15E may be specified for one type of biological sample.

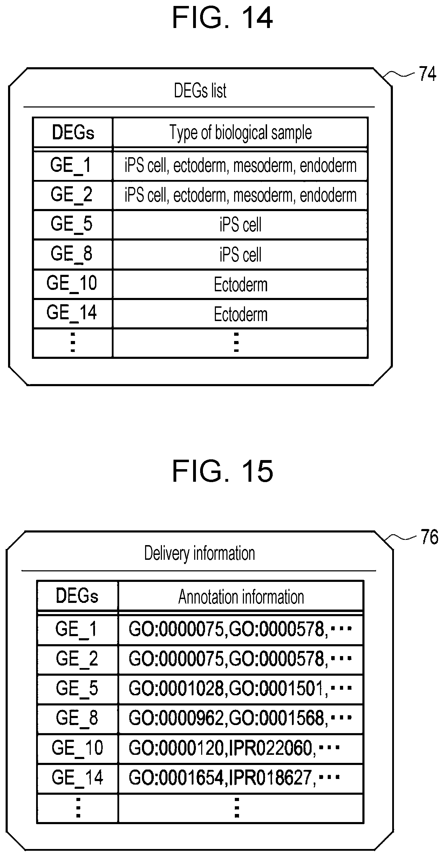

Fig. 14 illustrates the DEGs list 74 in which DEGs and the types of biological samples registered in the extraction targets 15E from which the DEGs are extracted are registered. For some DEGs, such as DEGs having ID "GE_5" and "GE_10", only one type of biological sample is registered, and for some DEGs, such as DEGs having ID "GE_1" and "GE_2", a plurality of types of biological samples including "iPS cell", "ectoderm", "mesoderm", and "endoderm" are registered. That is, some DEGs belong to only one type of biological sample, and some DEGs belong to a plurality of types of biological samples.

Fig. 15 illustrates the delivery information 76 that is information in which the DEGs and pieces of annotation information corresponding to each of the DEGs are registered.

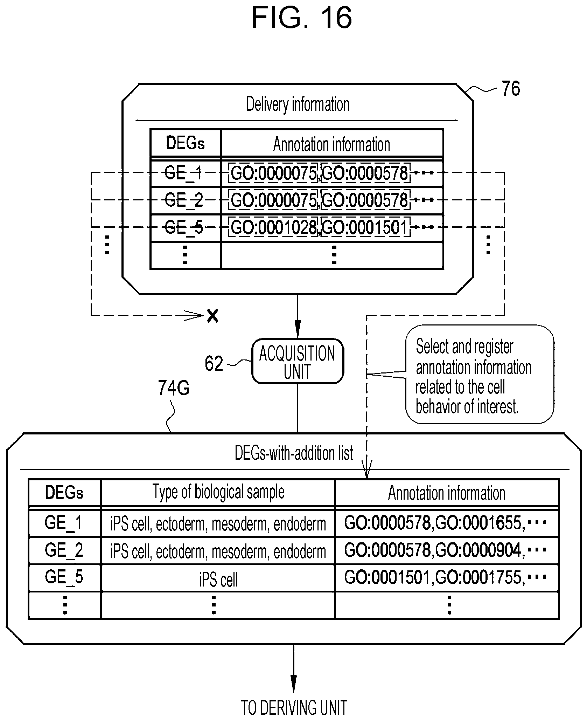

Fig. 16 illustrates the DEGs-with-addition list 74G that is created by adding an item "annotation information" to the DEGs list 74 illustrated in Fig. 14. With the DEGs-with-addition list 74G, the pieces of annotation information are associated with the types of biological samples.

The acquisition unit 62 selects pieces of annotation information related to the cell behavior of interest in the category and number range specifying information 70 from among the pieces of annotation information registered in the delivery information 76. The acquisition unit 62 registers only the selected pieces of annotation information in the DEGs list 74 to thereby create the DEGs-with-addition list 74G.

As illustrated in Fig. 9, "differentiation potency" is specified as the cell behavior of interest in this example. Accordingly, the acquisition unit 62 does not select, for example, pieces of annotation information having IDs "GO:0000075" and "GO:0001028" that are not related to differentiation potency but selects and registers, for example, only pieces of annotation information having IDs "GO:0000578" and "GO:0001501" that are related to differentiation potency. Note that a search keyword related to the cell behavior of interest may be included in the second delivery request 75, and the annotation information DB server 13 may select pieces of annotation information related to the cell behavior of interest.

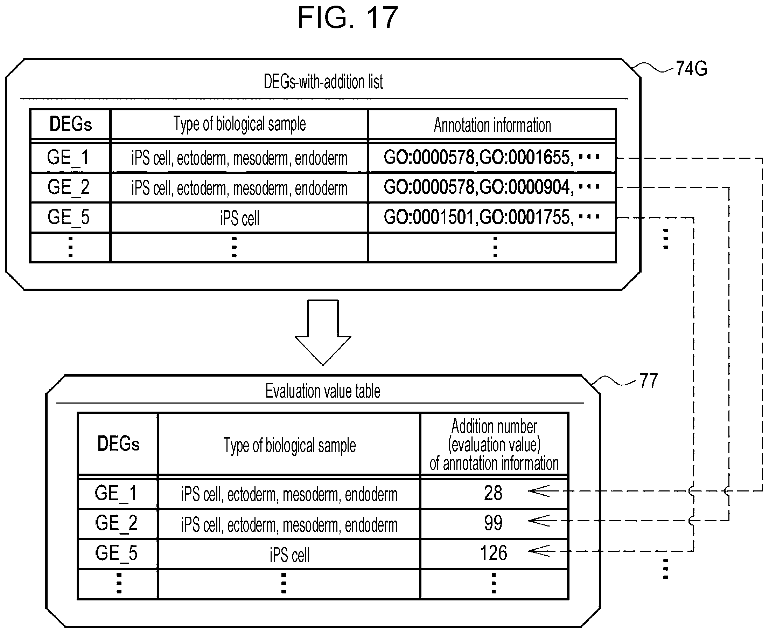

As illustrated in Fig. 17, the deriving unit 63 counts, on the basis of the DEGs-with-addition list 74G, an addition number that is the number of pieces of annotation information added to each of the DEGs. The counted addition numbers are registered as is in the evaluation value table 77 as evaluation values. For example, when 28 pieces of annotation information are added to a DEG having ID "GE_1", "28" that is equal to the addition number is registered in the evaluation value table 77 as the evaluation value.

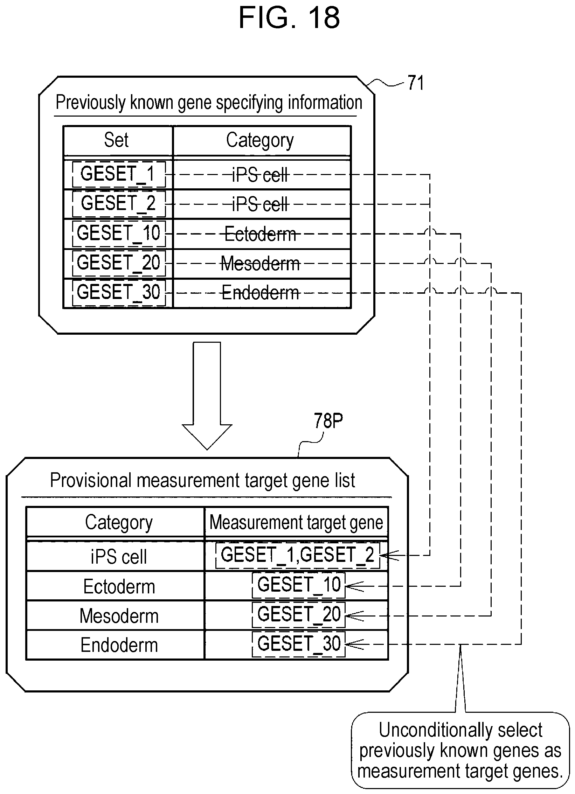

As illustrated in Fig. 18, the selection unit 64 first unconditionally selects the previously known gene sets specified in the previously known gene specifying information 71 as measurement target genes. Accordingly, a provisional measurement target gene list 78P in which the previously known gene sets are registered as measurement target genes is generated. This form in which the previously known gene sets are unconditionally selected as measurement target genes is an example of a form in which the weights of the evaluation values of the previously known genes are increased to make the previously known genes be always selected as measurement target genes.

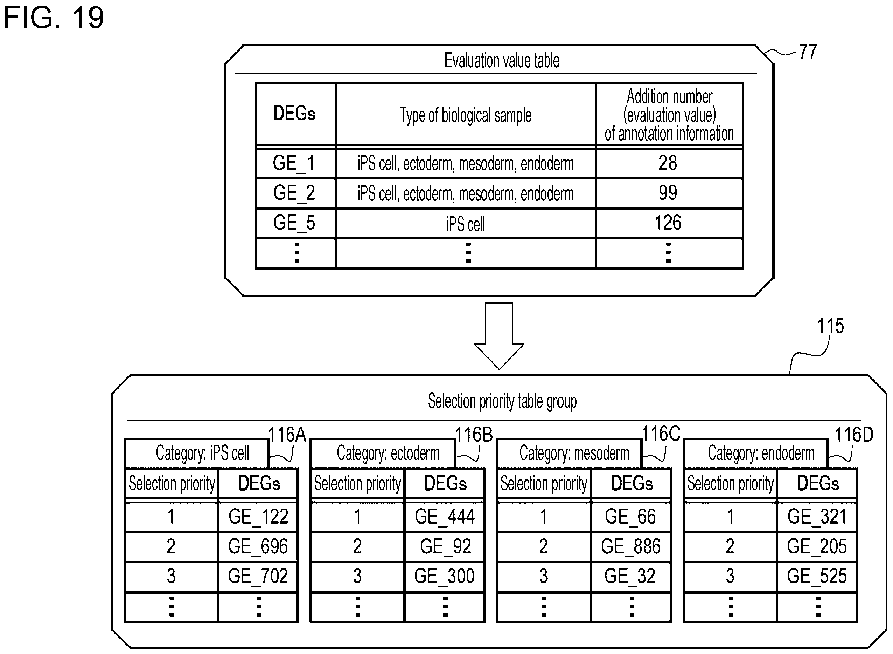

As illustrated in Fig. 19, the selection unit 64 generates a selection priority table group 115 on the basis of the evaluation value table 77. The selection priority table group 115 includes a selection priority table 116A for the category "iPS cell" corresponding to the type of biological sample "iPS cell", a selection priority table 116B for the category "ectoderm" corresponding to the type of biological sample "ectoderm", a selection priority table 116C for the category "mesoderm" corresponding to the type of biological sample "mesoderm", and a selection priority table 116D for the category "endoderm" corresponding to the type of biological sample "endoderm". The selection unit 64 assigns, for each of the categories, selection priorities to the DEGs in descending order of evaluation value (in descending order of the addition number, which is the number of pieces of added annotation information). That is, the selection priority of a DEG having the highest evaluation value is set to the first priority, the selection priority of a DEG having the second highest evaluation value is set to the second priority, the selection priority of a DEG having the third highest evaluation value is set to the third priority, and so on.

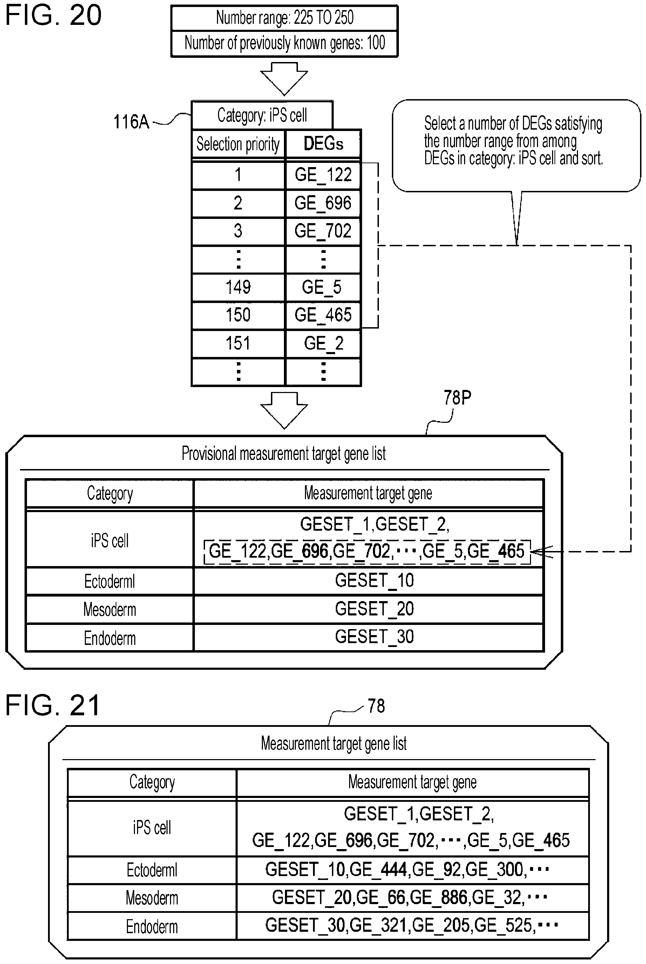

As illustrated in Fig. 20, the selection unit 64 selects with reference to the selection priority tables 116, a number of measurement target genes, the number satisfying a number range from among the DEGs prepared for each category and sorts the measurement target genes into the category.

Fig. 20 illustrates a state where measurement target genes belonging to the category "iPS cell" are selected from among the DEGs prepared for the category "iPS cell". Fig. 20 illustrates an example case where "225 to 250" illustrated in Fig. 9 is specified as the number range for the category "iPS cell" and the number of previously known genes belonging to the category "iPS cell" and selected as illustrated in Fig. 18 is 100. In this case, to satisfy the number range, 125 DEGs at the minimum and 150 DEGs at the maximum need to be selected. Accordingly, the selection unit 64 selects DEGs having the first to 150-th selection priorities, that is, 150 DEGs in total, from the selection priority table 116A. The selection unit 64 registers the selected 150 DEGs in the provisional measurement target gene list 78P as measurement target genes belonging to the category "iPS cell".

Although not illustrated, also for the other categories "ectoderm", "mesoderm", and "endoderm", the selection unit 64 similarly selects a number of DEGs, the number satisfying the number range, with reference to the selection priority tables 116B to 116D. The selection unit 64 registers the selected DEGs in the provisional measurement target gene list 78P as measurement target genes. The selection unit 64 thus selects measurement target genes sequentially to thereby generate the measurement target gene list 78 that satisfies the number range for each of the categories at the end as illustrated in Fig. 21.

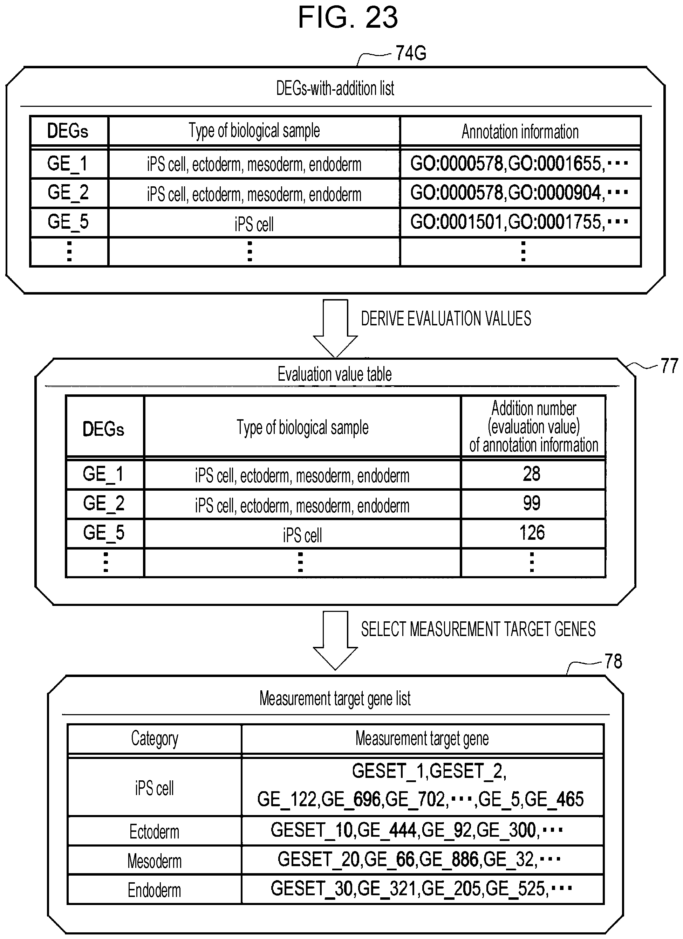

Fig. 22 and Fig. 23 are diagrams summarizing the series of processes performed by the extraction unit 61, the acquisition unit 62, the deriving unit 63, and the selection unit 64. First, as illustrated in Fig. 22, the extraction unit 61 extracts DEGs from the extraction targets 15E and generates the DEGs list 74. The acquisition unit 62 acquires pieces of annotation information by acquiring the delivery information 76 from the annotation information DB server 13. The acquisition unit 62 adds the pieces of annotation information in the delivery information 76 to the DEGs list 74 to thereby create the DEGs-with-addition list 74G.

As illustrated in Fig. 23, the deriving unit 63 counts the addition number, which is the number of pieces of annotation information added to each of the DEGs, and registers the addition numbers in the evaluation value table 77 as evaluation values. The selection unit 64 selects measurement target genes on the basis of the evaluation values and generates the measurement target gene list 78.

Fig. 24 illustrates the measurement target gene display screen 120 on which the measurement target genes registered in the measurement target gene list 78 are displayed. On the measurement target gene display screen 120, display regions 121A, 121B, 121C, and 121D are provided for the respective categories. In the display region 121A, measurement target genes belonging to the category "iPS cell" are displayed. Measurement target genes belonging to the category "ectoderm" are displayed in the display region 121B, measurement target genes belonging to the category "mesoderm" are displayed in the display region 121C, and measurement target genes belonging to the category "endoderm" are displayed in the display region 121D.

Below the measurement target gene display screen 120, a save button 122, a print button 123, and an OK button 124 are provided. The save button 122 is selected to save the measurement target gene list 78 in the storage device 45. The print button 123 is selected to print the measurement target gene list 78. In response to selection of the OK button 124, the display control unit 65 hides the displayed measurement target gene display screen 120.

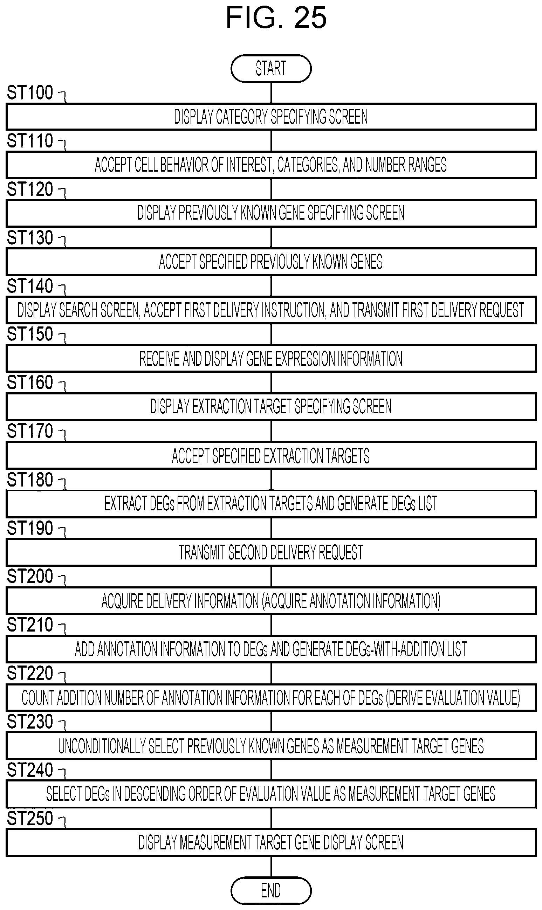

Now, operations based on the above-described configuration will be described with reference to the flowchart in Fig. 25. First, in response to the start of the operation program 55 in the information processing apparatus 10, the CPU 47 of the information processing apparatus 10 functions as the instruction accepting unit 60, the extraction unit 61, the acquisition unit 62, the deriving unit 63, the selection unit 64, and the display control unit 65 as illustrated in Fig. 8.

Under the control of the display control unit 65, the category specifying screen 80 illustrated in Fig. 9 is displayed on the display 49 (step ST100). The user inputs a cell behavior of interest and desired categories and number ranges and selects the enter button 86. Accordingly, the instruction accepting unit 60 accepts the specified cell behavior of interest, categories, and number ranges (step ST110) and generates the category and number range specifying information 70. The category and number range specifying information 70 is output to the selection unit 64 from the instruction accepting unit 60.

Subsequently, under the control of the display control unit 65, the previously known gene specifying screen 95 illustrated in Fig. 12 is displayed on the display 49 (step ST120). The user inputs desired previously known gene sets and selects the enter button 98. Accordingly, the instruction accepting unit 60 accepts the specified previously known gene sets (step ST130) and generates the previously known gene specifying information 71. The previously known gene specifying information 71 is output to the selection unit 64 from the instruction accepting unit 60.

Under the control of the display control unit 65, the search screen not illustrated is displayed on the display 49. The instruction accepting unit 60 accepts the first delivery instruction including search keywords and given by the user. Accordingly, the first delivery request 72 including the search keywords is transmitted to the gene expression information DB server 12 from the instruction accepting unit 60 (step ST140).

In response to the first delivery request 72, pieces of gene expression information 15 are delivered from the gene expression information DB server 12. The pieces of gene expression information 15 are input to the display control unit 65. Under the control of the display control unit 65, the display screen not illustrated for the pieces of gene expression information 15 is displayed on the display 49 (step ST150).

Under the control of the display control unit 65, the extraction target specifying screen 105 illustrated in Fig. 13 is displayed on the display 49 (step ST160). The user inputs desired extraction targets 15E and selects the enter button 108. Accordingly, the instruction accepting unit 60 accepts the specified extraction targets 15E (step ST170) and generates the extraction target specifying information 73. The extraction target specifying information 73 is transmitted to the extraction unit 61 from the instruction accepting unit 60.

The extraction unit 61 extracts DEGs from the extraction targets 15E and generates the DEGs list 74 illustrated in Fig. 14 (step ST180). The DEGs list 74 is output to the acquisition unit 62 from the extraction unit 61. Subsequently, the second delivery request 75 based on the DEGs list 74 is transmitted to the annotation information DB server 13 from the acquisition unit 62 (step ST190).

In response to the second delivery request 75, the delivery information 76 including pieces of annotation information illustrated in Fig. 15 is delivered from the annotation information DB server 13. The delivery information 76 is input to the acquisition unit 62. Accordingly, the acquisition unit 62 acquires the delivery information 76, that is, the pieces of annotation information (step ST200). Step ST200 is an example of "acquisition process" in the technique of the present disclosure.

As illustrated in Fig. 16, the acquisition unit 62 adds pieces of annotation information to the DEGs list 74 on the basis of the delivery information 76 to thereby create the DEGs-with-addition list 74G from the DEGs list 74 (step ST210). At this time, only pieces of annotation information related to the cell behavior of interest are selected and added. The DEGs-with-addition list 74G is output to the deriving unit 63 from the acquisition unit 62.

As illustrated in Fig. 17, the deriving unit 63 counts the addition number, which is the number of pieces of annotation information added to each of the DEGs, and registers the addition numbers in the evaluation value table 77 as evaluation values (step ST220). The evaluation value table 77 is output to the selection unit 64 from the deriving unit 63. Step ST220 is an example of "deriving process" in the technique of the present disclosure.

As illustrated in Fig. 18, the selection unit 64 unconditionally selects previously known genes as measurement target genes (step ST230).

As illustrated in Fig. 20, the selection unit 64 selects a number of DEGs, the number satisfying the number range, from among the DEGs prepared for each category in descending order of evaluation value. The selected DEGs are sorted into the category as measurement target genes (step ST240). As a result of the above-described steps, the measurement target gene list 78 illustrated in Fig. 21 is generated. The measurement target gene list 78 is output to the display control unit 65 from the selection unit 64. Step ST240 is an example of "selection process" in the technique of the present disclosure.

Last, the display control unit 65 displays the measurement target gene display screen 120 illustrated in Fig. 24 on the display 49 (step ST250). The user checks the measurement target genes on the measurement target gene display screen 120.

As described above, the information processing apparatus 10 includes the acquisition unit 62, the deriving unit 63, and the selection unit 64. The acquisition unit 62 acquires pieces of annotation information added to each of a plurality of genes. The deriving unit 63 calculates an evaluation value of each of the plurality of genes on the basis of the pieces of annotation information. The selection unit 64 selects measurement target genes from among the plurality of genes on the basis of the evaluation values. Accordingly, measurement target genes can be selected in a data-driven manner, which is backed by the evaluation values based on the pieces of annotation information. The measurement target genes thus selected can be easily deployed in a multilevel manner and are customized so as to be suitable to the study target cell. Therefore, more appropriate measurement target genes that lead to elucidation of cell behaviors can be selected.

The acquisition unit 62 selects pieces of annotation information related to a cell behavior of interest. The selection unit 64 derives evaluation values on the basis of only the selected pieces of annotation information. Accordingly, measurement target genes can be selected on the basis of only the pieces of annotation information specifically related to the cell behavior of interest. In other words, pieces of annotation information that are less likely to be related to the cell behavior of interest are eliminated as noise, and measurement target genes can be selected by using only pieces of annotation information that are highly likely to be related to the cell behavior of interest.

The acquisition unit 62 adds, with reference to the annotation information DB 16 in which pieces of annotation information for genes are registered, pieces of annotation information to genes. Accordingly, pieces of annotation information can be easily added by using the existing annotation information DB 16.

With the pieces of annotation information, the types of biological samples are associated. The instruction accepting unit 60 accepts a plurality of categories defined in accordance with the types of biological samples and a number range for each of the plurality of categories, the plurality of categories and the number ranges being specified by the user. The selection unit 64 selects a number of genes, the number satisfying the number range, from among genes prepared for each category of the plurality of categories and sorts the selected genes into the category as measurement target genes. Accordingly, an appropriate number of measurement target genes can be selected for each of the categories.

The categories include "iPS cell", "ectoderm", "mesoderm", and "endoderm". Accordingly, measurement target genes for each of the categories related to the iPS cell 25 that has been increasingly drawing attention recently can be acquired. Note that to measure the expression levels of genes for the purpose of evaluating iPS cells and their differentiation process, the categories preferably include "iPS cell", "ectoderm", "mesoderm", and "endoderm" described above. However, to measure the expression levels of genes for a purpose other than the above-described purpose, the categories are not limited to "iPS cell", "ectoderm", "mesoderm", and "endoderm" described above.

The deriving unit 63 counts the addition number, which is the number of pieces of added annotation information, for each of the plurality of genes and derives evaluation values on the basis of the addition numbers. Accordingly, the evaluation values can be easily derived.

The genes include previously known genes. The instruction accepting unit 60 accepts previously known genes specified by the user. As one form for increasing the weights of the evaluation values of previously known genes, the selection unit 64 unconditionally selects previously known genes as measurement target genes. Accordingly, the user's intention to measure previously known genes can be reflected. Further, previously known genes that are acquired as a result of past findings can be effectively adopted as measurement target genes.

The selection unit 64 selects more than 100 and less than or equal to 1000 measurement target genes. 100 or less measurement target genes are not sufficient for elucidation of cell behaviors. In contrast, more than 1000 measurement target genes lead to an increased test time and increased test costs and make deployment to multilevel experiments difficult.

The genes include DEGs. Accordingly, measurement target genes that are considered to contribute to elucidation of cell behaviors to a larger degree can be selected.

Although a form in which previously known genes are unconditionally selected as measurement target genes has been employed, the form need not be employed. Similarly to DEGs, previously known genes may also be selected on the basis of evaluation values derived from acquired pieces of annotation information. At this time, the weights of the evaluation values of the previously known genes may be made larger than those of DEGs. In this case, a degree of importance may be set for each of the previously known genes and evaluation values may be derived by taking into consideration the degrees of importance. Specifically, as the degree of importance is higher, a higher evaluation value is derived. Note that genes other than the previously known genes, that is, for example, DEGs, may be assumed to have the lowest degree of importance, and evaluation values may be derived.

Previously known genes need not be specified. For example, for a new study target cell for which previously known genes do not exist, previously known genes need not be specified.

The extraction targets 15E need not be specified, and all pieces of gene expression information 15 delivered from the gene expression information DB server 12 may be assumed to be the extraction targets 15E.

Categories need not be specified. Even when categories are not specified, the range of the number of selected measurement target genes or at least the upper limit needs to be specified.

The gene expression information DB 14 is not limited to a public DB, such as GEO described above. For example, the gene expression information DB 14 may be, for example, a local DB in which pieces of gene expression information 15 acquired as a result of measurement performed at a laboratory to which the user belongs are registered. The annotation information DB 16 is similarly not limited to a public DB, such as DAVID or InterPro, and may be, for example, a local DB prepared by a laboratory to which the user belongs.

### Second Embodiment

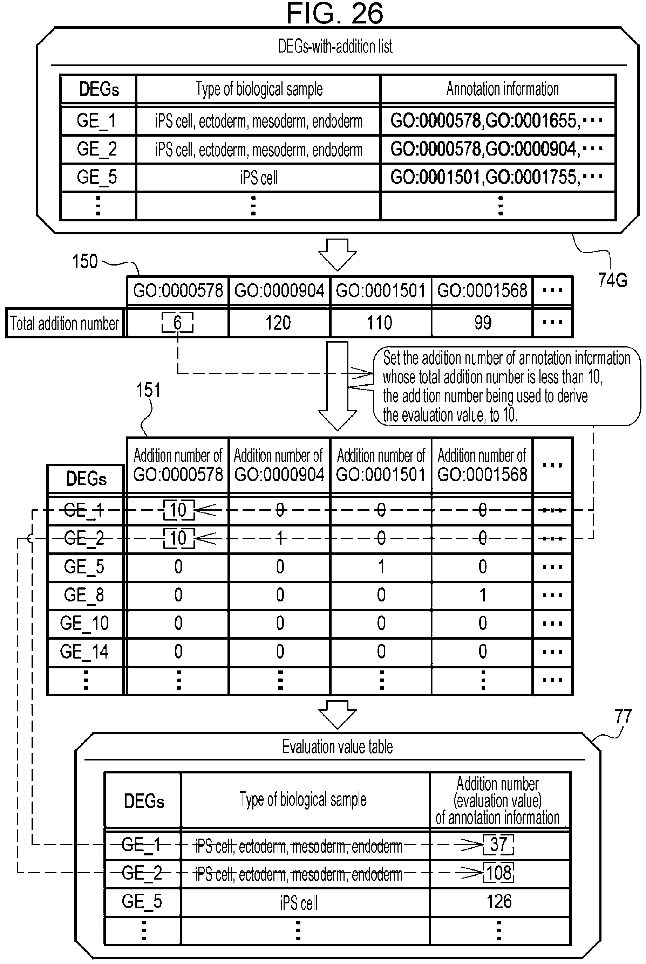

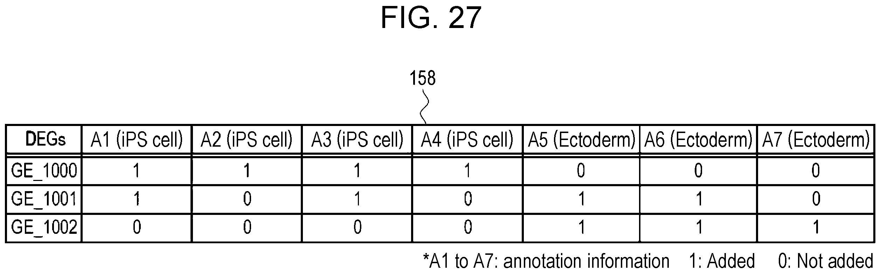

In a second embodiment illustrated in Fig. 26 and Fig. 27, weights are assigned to evaluation values in accordance with the information values of pieces of annotation information.

Fig. 26 illustrates an example where a piece of annotation information whose addition number is relatively small, that is, a piece of annotation information that has a relatively high rarity, is determined to have a high information value and the addition number of the piece of annotation information is increased. The deriving unit 63 first counts, on the basis of the DEGs-with-addition list 74G, an addition number (hereinafter referred to as "total addition number") of each of the pieces of annotation information added to the DEGs as shown by a table 150. The deriving unit 63 compares each total addition number with a preset threshold value. The deriving unit 63 determines a piece of annotation information whose total addition number is less than the threshold value to have a high information value and sets the addition number, of the piece of annotation information, used to derive the evaluation value to a value greater than 1 as shown by a table 151. That is, the deriving unit 63 increases the weight of the piece of annotation information that is determined to have a high information value. The deriving unit 63 counts the addition number, which is the number of pieces of annotation information added to each of the DEGs, including the addition number assigned a weight and generates the evaluation value table 77.

Fig. 26 illustrates an example case where "10" is set as the threshold value and the addition number of the piece of annotation information having ID "GO:0000578" whose total addition number is "6" and less than the threshold value is increased to "10".

Fig. 27 illustrates an example where weights are assigned to evaluation values on the basis of the orthogonality of pieces of annotation information. The deriving unit 63 determines a set of genes that can cover pieces of annotation information without omission and without duplication to the extent possible to have high orthogonality.

A table 158 shows the states of addition of pieces of annotation information A1 to A7 to three DEGs having IDs "GE_1000", "GE_1001", and "GE_1002". Among the pieces of annotation information A1 to A7, "iPS cell" is associated with the pieces of annotation information A1 to A4 as the type of biological sample, and "ectoderm" is associated with the pieces of annotation information A5 to A7 as the type of biological sample.

In this case, from the viewpoint of only the addition numbers, which is the number of pieces of added annotation information, the DEGs having ID "GE_1000" and ID "GE _1001" are given priority over the DEG having ID "GE_1002" and selected as measurement target genes. However, taking into consideration the orthogonality of the pieces of annotation information, the DEG having ID "GE_1002" is given priority over the DEG having ID "GE_1001" and selected as a measurement target gene. Accordingly, when the DEGs having ID "GE_1000" and ID "GE_1002" are selected as measurement target genes at the end, both "iPS cell" and "ectoderm" can be covered.

Note that evaluation values may be derived on the basis of the number of pieces of annotation information that can be covered by a combination with another gene. With reference to, for example, the table 158, by the combination of the DEGs having ID "GE_1000" and ID "GE_1001", six pieces of annotation information can be covered. By the combination of the DEGs having ID "GE_1000" and ID "GE_1002", seven pieces of annotation information can be covered. By the combination of the DEGs having ID "GE_1001" and ID "GE_1002", five pieces of annotation information can be covered. From these results, the evaluation values of the DEGs having ID "GE_1000" and ID "GE_1002" are made higher than the evaluation value of the DEG having ID "GE_1001".

As described above, in the second embodiment, the deriving unit 63 assigns weights to the evaluation values in accordance with the information values of the pieces of annotation information. Accordingly, when, for example, the weight of the addition number of a piece of annotation information that is determined to have a high information value is increased, genes to which the piece of annotation information considered to have a high information value is added are more likely to be selected as measurement target genes. Therefore, the measurement target genes can be more appropriate and reliable.

In Fig. 26, the deriving unit 63 determines a piece of annotation information having a relatively high rarity to have a high information value and increases the weight. Accordingly, a gene to which a rare piece of annotation information that is likely to be overlooked is added can be selected as a measurement target gene.

In Fig. 27, the deriving unit 63 assigns weights to the evaluation values on the basis of the orthogonality of the pieces of annotation information. Accordingly, a set of genes that can cover pieces of annotation information without omission and without duplication to the extent possible can be selected as measurement target genes.

The examples in Fig. 26 and Fig. 27 may be combined and employed. In this case, for example, the evaluation value of a DEG to which a piece of annotation information whose total addition number is less than the threshold value is added, the piece of annotation information having high orthogonality, is increased by 100.

Although a piece of annotation information having a relatively high rarity is determined to be a piece of annotation information having a high information value in the example in Fig. 26, a piece of annotation information having a high information value is not limited to this example. For example, a piece of annotation information that is inserted in a relatively large number of research papers may be determined to be a piece of annotation information having a high information value.

Although a weight is assigned to the addition number of a piece of annotation information added to the DEGs in Fig. 26, the weighting is not limited to this. When evaluation values are derived also for previously known genes, a weight may be assigned to the addition number of a piece of annotation information added to the previously known genes as in the case illustrated in Fig. 26. The form illustrated in Fig. 27 may also be applied to previously known genes.

### Third Embodiment

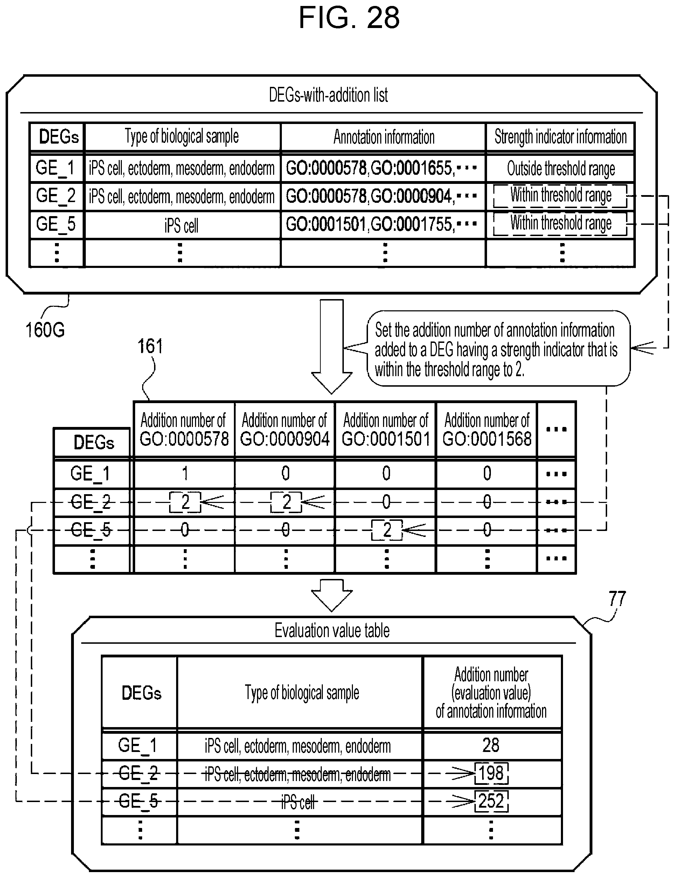

In a third embodiment illustrated in Fig. 28, the weight of the evaluation value of a gene having a strength indicator that is within a preset threshold range is increased.

Fig. 28 illustrates a DEGs-with-addition list 160G of a third embodiment that includes an item "strength indicator information". In the item "strength indicator information", whether the strength indicator is within the preset threshold range is registered. The strength indicator is, for example a fold-change or a q-value that indicates a significant difference in expression subjected to multiple-testing corrections.

The deriving unit 63 sets the addition number of a piece of annotation information added to a DEG having a strength indicator that is within the threshold range, the addition number being used to derive the evaluation value, to a value greater than 1 as shown by a table 161. That is, the deriving unit 63 increases the weight of the evaluation value of a DEG having a strength indicator that is within the threshold range. The deriving unit 63 counts the addition number, which is the number of pieces of annotation information added to each of the DEGs, including the addition number assigned a weight and generates the evaluation value table 77.

Fig. 28 illustrates an example case where the strength indicators of, for example, the DEGs having IDs "GE_2" and "GE_5" are within the threshold range and the addition numbers of pieces of annotation information added to these DEGs are set to "2".

As described above, in the third embodiment, the deriving unit 63 increases the weight of the evaluation value of a DEG having a strength indicator that is within the threshold range. Accordingly, a DEG that has a strength indicator within the threshold range and that is considered to be more important to elucidate the characteristics of the biological sample can be selected as a measurement target gene. Note that the second embodiment and the third embodiment may be combined and employed.

### Fourth Embodiment

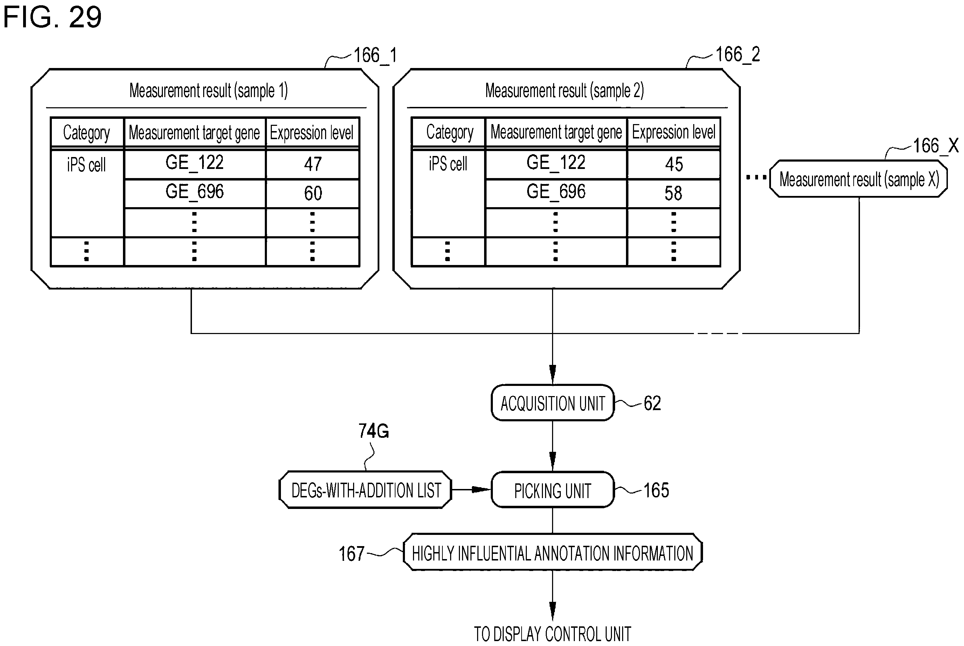

In a fourth embodiment illustrated in Fig. 29 to Fig. 34, measurement results 166 regarding measurement target genes are acquired. On the basis of the measurement results 166, pieces of annotation information (hereinafter referred to as "pieces of highly influential annotation information") 167 that influence cell behaviors to a relatively large degree are picked with a statistical method from among pieces of annotation information 171 added to the measurement target genes, and the picked pieces of highly influential annotation information 167 are presented to the user.

As illustrated in Fig. 29, the CPU 47 of the information processing apparatus 10 of the fourth embodiment functions as a picking unit 165 as well as the processing units 60 to 65 (only the acquisition unit 62 is illustrated in Fig. 29) illustrated in Fig. 8.

The acquisition unit 62 acquires a plurality of measurement results 166_1, 166_2, ..., and 166_X. The measurement results 166_1 to 166_X are, for example, the results of actually measuring the expression levels of the measurement target genes in the stage of the iPS cell 25 for a plurality of samples 1, 2, ..., X showing a low efficiency of inducing differentiation from the iPS cell 25 into the tissue cells 30. The measurement results 166_1 to 166_X are transmitted to the information processing apparatus 10 from, for example, a measurement device that measures the expression levels of genes and are input to the acquisition unit 62. The acquisition unit 62 outputs the measurement results 166_1 to 166_X to the picking unit 165.

The picking unit 165 picks the pieces of highly influential annotation information 167 on the basis of the measurement results 166_1 to 166_X from the acquisition unit 62 and the DEGs-with-addition list 74G. The picking unit 165 outputs the pieces of highly influential annotation information 167 to the display control unit 65.

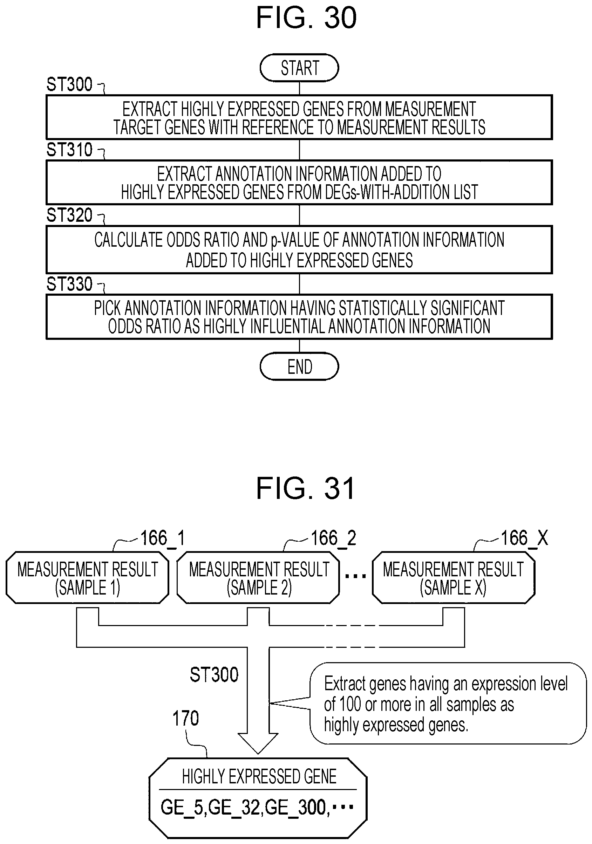

Fig. 30 to Fig. 33 illustrate a processing procedure for picking the pieces of highly influential annotation information 167 by the picking unit 165. First, the picking unit 165 extracts highly expressed genes 170 from the measurement target genes with reference to the measurement results 166_1 to 166_X, as illustrated in step ST300 in Fig. 30 and in Fig. 31. The highly expressed genes 170 are, for example, measurement target genes each having an expression level that is greater than or equal to a threshold value in all samples 1 to X. Fig. 31 illustrates an example case where "100" is set as the threshold value and measurement target genes having IDs "GE_5", "GE_32", "GE_300", and so on are extracted as the highly expressed genes 170.

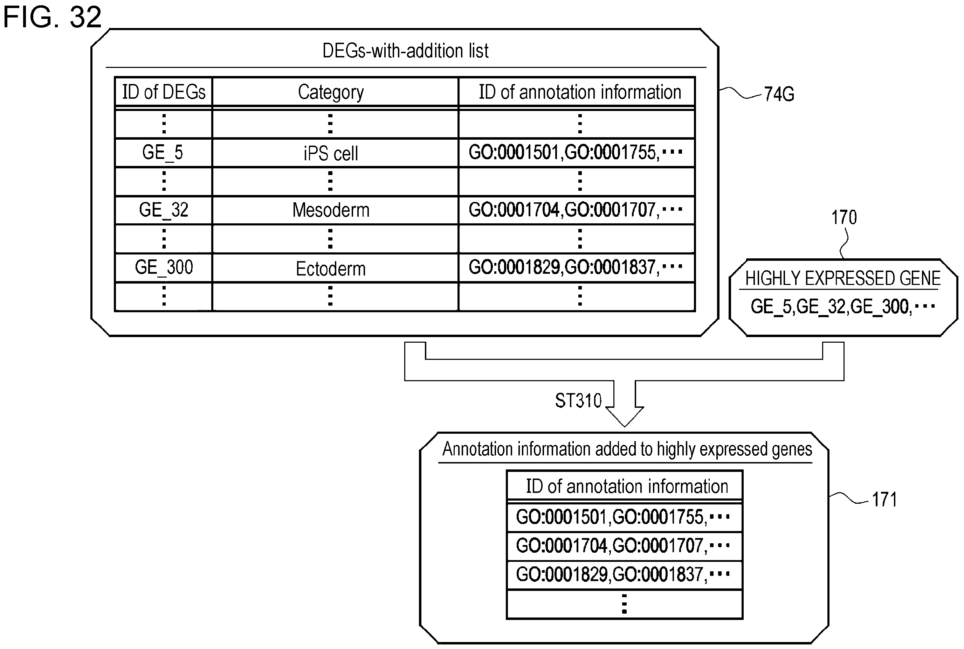

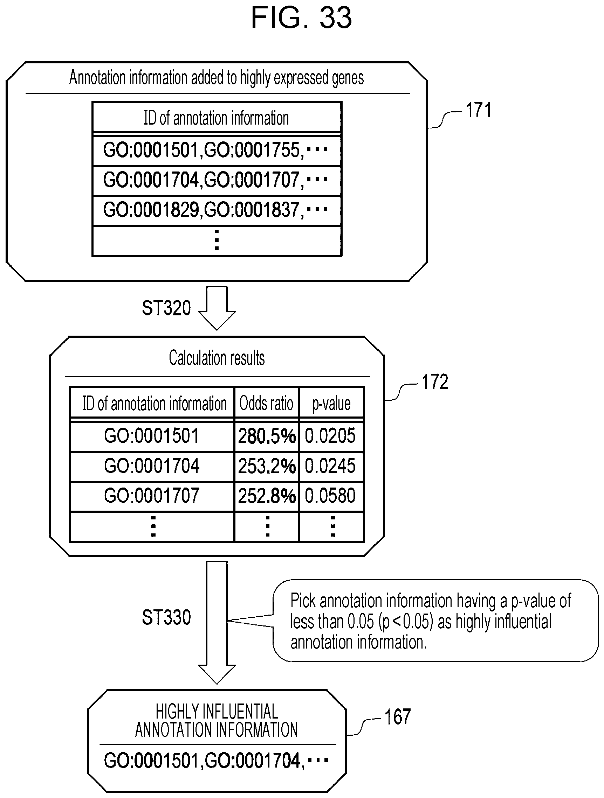

Next, the picking unit 165 extracts pieces of annotation information 171 added to the highly expressed genes 170 from the DEGs-with-addition list 74G as illustrated in step ST310 in Fig. 30 and in Fig. 32. Subsequently, the picking unit 165 calculates the odds ratio and the p-value for each of the pieces of annotation information 171 added to the highly expressed genes 170 as illustrated in step ST320 in Fig. 30 and in calculation results 172 in Fig. 33. Last, the picking unit 165 picks as the pieces of highly influential annotation information 167, pieces of annotation information 171 that each have a p-value of less than 0.05 and that are statistically significant from among the pieces of annotation information 171 added to the highly expressed genes 170, as illustrated in step ST330 in Fig. 30 and in a part below the calculation results 172 in Fig. 33. Fig. 33 illustrates an example case where a piece of annotation information 171 having ID "GO:0001501" and having a p-value "0.0205", a piece of annotation information 171 having ID "GO:0001704" and having a p-value "0.0245", and so on are picked as the pieces of highly influential annotation information 167.

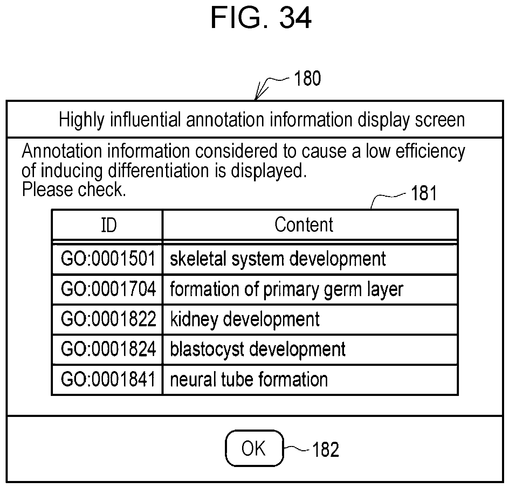

Fig. 34 illustrates a highly influential annotation information display screen 180 that is displayed on the display 49 under the control of the display control unit 65. On the highly influential annotation information display screen 180, a display region 181 for the pieces of highly influential annotation information 167 is provided. In the display region 181, the list of the highly influential annotation information 167 and their content is displayed. In response to selection of an OK button 182, the display control unit 65 hides the highly influential annotation information display screen 180.

As described above, in the fourth embodiment, the acquisition unit 62 acquires the measurement results 166 regarding measurement target genes. The picking unit 165 picks, on the basis of the measurement results 166, the pieces of highly influential annotation information 167 that influence cell behaviors to a relatively large degree from among the pieces of annotation information 171 added the measurement target genes, with a statistical method. The display control unit 65 displays the highly influential annotation information display screen 180 on the display 49 to thereby present the pieces of highly influential annotation information 167 to the user. Accordingly, the user can deduce, for example, major causes of a low efficiency of inducing differentiation on the basis of the pieces of highly influential annotation information 167 and utilize the result of deduction to the next culture. The pieces of highly influential annotation information 167 are picked with a statistical method, and therefore, for example, major causes of a low efficiency of inducing differentiation can be correctly deducted.

Note that in addition to the pieces of highly influential annotation information 167, genes to which the pieces of highly influential annotation information 167 are added may be displayed on the highly influential annotation information display screen 180.

### Example

An example in a case where "differentiation potency" of the iPS cell 25 is selected as the cell behavior of interest as in the case in Fig. 9 will be described. The categories and the number range are the same as the examples illustrated in Fig. 9. That is, an example where "iPS cell", "ectoderm", "mesoderm", and "endoderm" are specified as the categories and "225 to 250" is specified for each of the categories as the number range will be described.

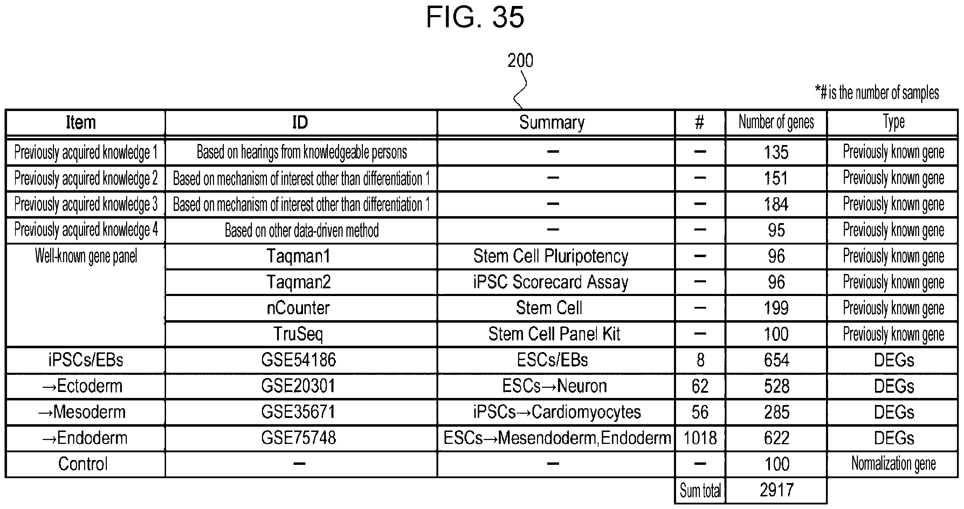

Fig. 35 is a table 200 showing previously known genes specified in order to select measurement target genes in this example and extracted DEGs. The previously known genes include those based on hearings from knowledgeable persons and well-known gene panels including TaqMan Scorecard. The DEGs include the iPS cell 25 or ES cells (Embryonic Stem cells) and those extracted from the extraction targets 15E in an experiment in which the iPS cell 25 or ES cells were made to differentiate into the three germ layers 26 or the tissue cells 30. In this example, from among about 2900 genes (some are duplicated) including those described above, about 1000 (specifically, 980) measurement target genes satisfying the number range were selected. More specifically, only pieces of annotation information related to differentiation potency were selected from among pieces of annotation information acquired from the annotation information DB 16 and were added to the previously known genes and the DEGs. On the basis of the pieces of annotation information, evaluation values were derived and a number of previously known genes and DEGs, the number satisfying the number range, were selected in the descending order of evaluation value. Apart from the previously known genes and DEGs, normalization genes were also selected as measurement target genes. The about 1000 measurement target genes thus selected are hereinafter referred to as C1000.

In the experiment of inducing differentiation from the iPS cell 25 into cardiac muscle cells, the expression levels of C1000 in 15 samples in the stage of the iPS cell 25 were measured. Of the 15 samples, 10 samples showed a high efficiency of inducing differentiation and 5 samples showed a low efficiency of inducing differentiation.

To verify the effect of the technique of the present disclosure, the expression levels of (about 21000) comprehensive genes were also measured separately with a microarray, for the iPS cell 25 before induced to differentiate in the 15 samples, as a comparative example.

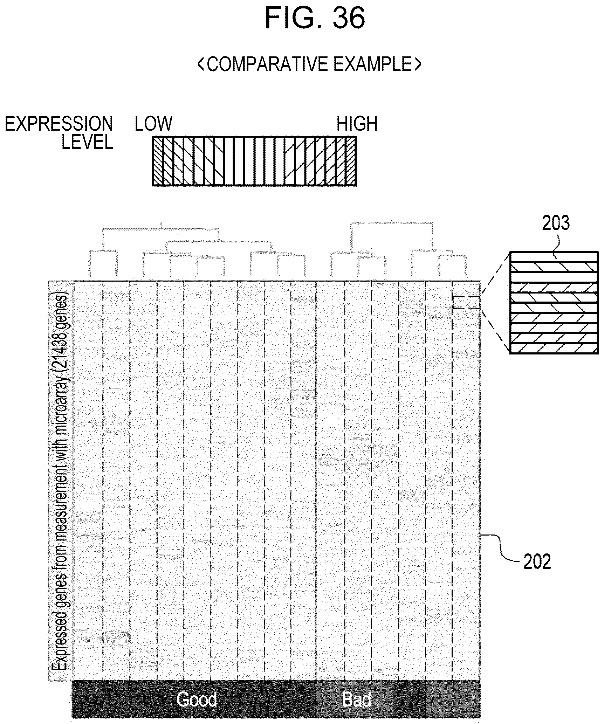

Fig. 36 illustrates measurement results 202 of the expression levels of the microarray. Each bar 203 indicates the expression level of a gene. According to the measurement results 202, the 15 samples were divided into a group of nine samples on the left side and a group of six samples on the right side by clustering, and the group of six samples included all five samples indicated as "Bad" and showing a low efficiency of inducing differentiation. That is, according to the measurement results 202 of the expression levels of the microarray, it was found that the efficiency of inducing differentiation (high or low) could be predicted with relatively high accuracy (the detection sensitivity of samples showing a low efficiency of inducing differentiation is 100% and the degree of specificity of samples showing a low efficiency of inducing differentiation is 83%) in the stage of the iPS cell 25. Note that "Good" indicates samples showing a high efficiency of inducing differentiation.

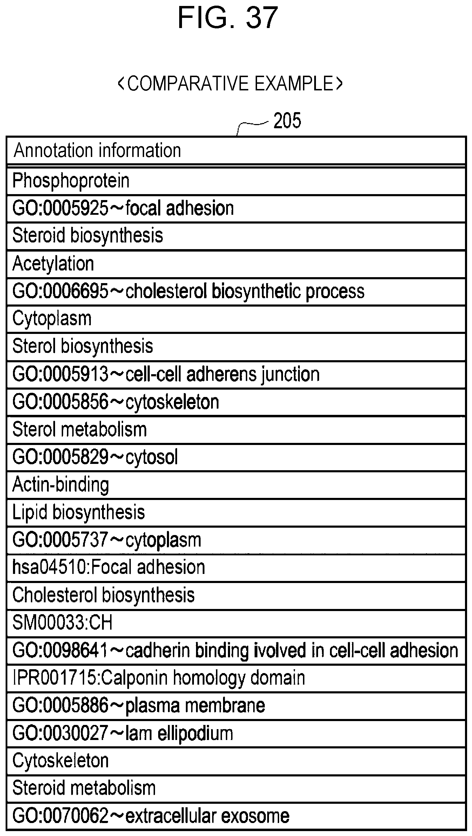

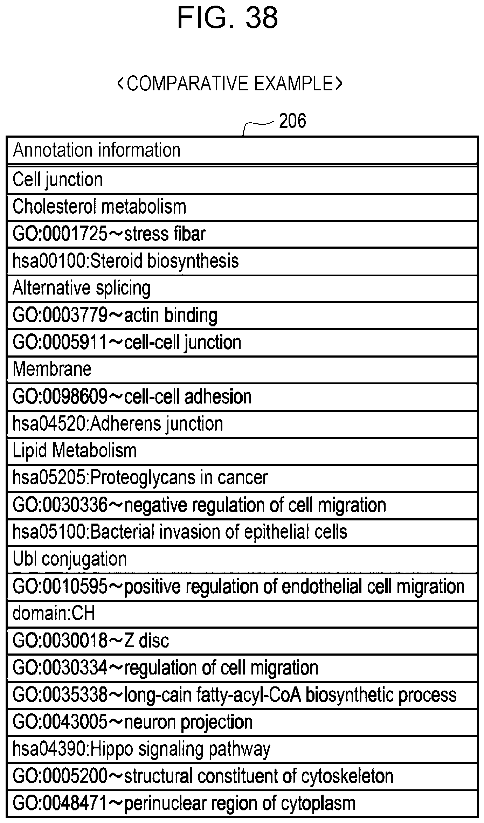

From among the genes used in measurement with the microarray, the highly expressed genes 170 were extracted and the pieces of highly influential annotation information 167 were further picked as in the fourth embodiment described above. The results are shown by a table 205 in Fig. 37 and a table 206 in Fig. 38. According to the table 205 and the table 206, it was found that various miscellaneous pieces of annotation information were picked as the pieces of highly influential annotation information 167 and it was difficult to acquire effective knowledge leading to elucidation of cell behaviors.

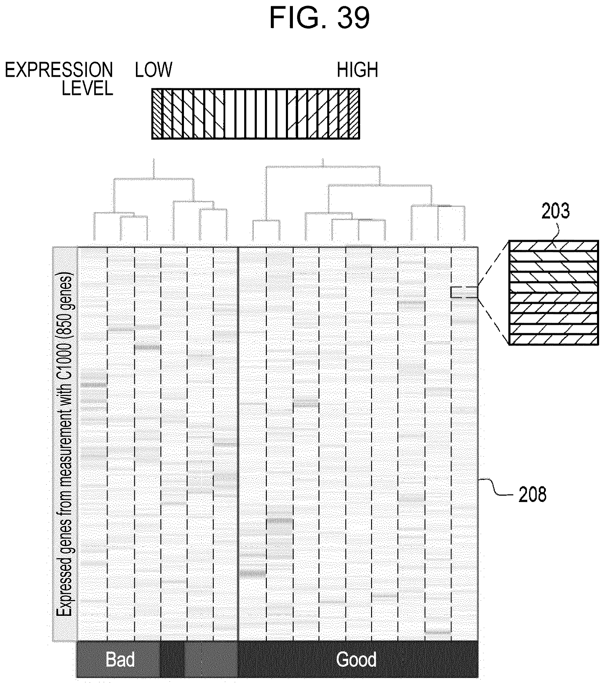

Fig. 39 illustrates measurement results 208 of the expression levels of C1000 measured for the iPS cell 25 before induced to differentiate in the 15 samples. According to the measurement results 208, the 15 samples were divided into a group of nine samples on the right side and a group of six samples on the left side by clustering, and the group of six samples included all five samples indicated as "Bad" and showing a low efficiency of inducing differentiation (the detection sensitivity of samples showing a low efficiency of inducing differentiation is 100% and the degree of specificity of samples showing a low efficiency of inducing differentiation is 83%). Therefore, it was verified with C1000 based on the technique of the present disclosure that the efficiency of inducing differentiation could be predicted at a level equivalent to the level attained by comprehensive measurement with the microarray.

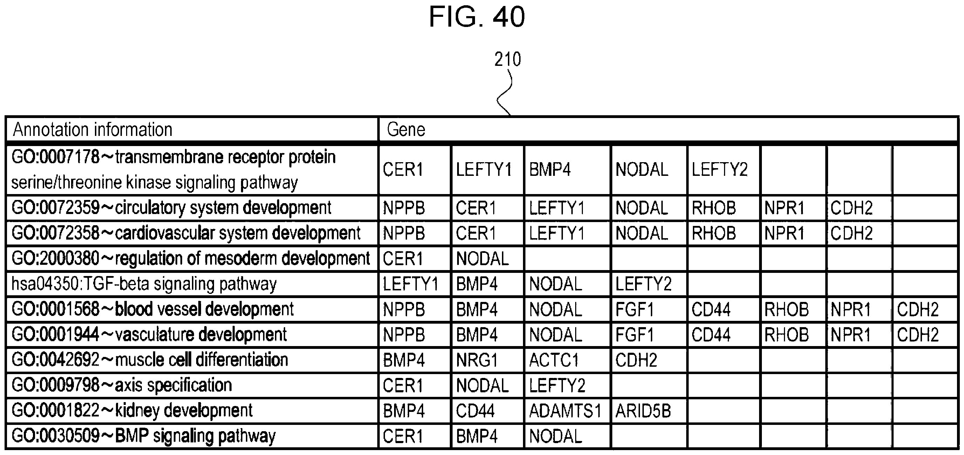

The highly expressed genes 170 were extracted from C1000 and the pieces of highly influential annotation information 167 were further picked as in the fourth embodiment described above. The results are shown by a table 210 in Fig. 40. According to the table 210, it is found that, specifically, a large number of pieces of annotation information regarding expression of blood-vessel-formation functions are picked. Further, genes including NODAL, LEFTY1, LEFTY2, CER1, and BMP4 are prominent, and these genes are considered to be likely to determine the efficiency of inducing differentiation (high or low). That is, it was verified that the technique of the present disclosure in which evaluation values are derived from pieces of annotation information and measurement target genes are selected on the basis of the evaluation values would be very useful in elucidating the characteristics of the biological sample.

Subsequently, the analysis capability of the set of measurement genes C1000 selected with the technique of the present disclosure and that of a set of measurement genes of TaqMan Scorecard, which is an existing representative method, were compared with each other. The measurement results acquired from the set of measurement genes of TaqMan Scorecard were created by using a simulation, from the measurement results of the expression levels of comprehensive genes with the microarray while extracting 84 genes of TaqMan Scorecard.

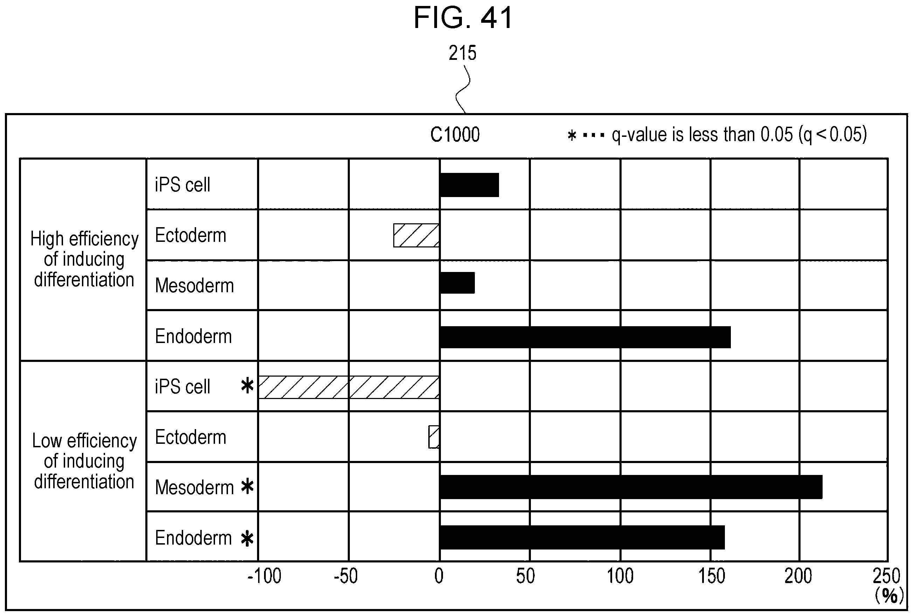

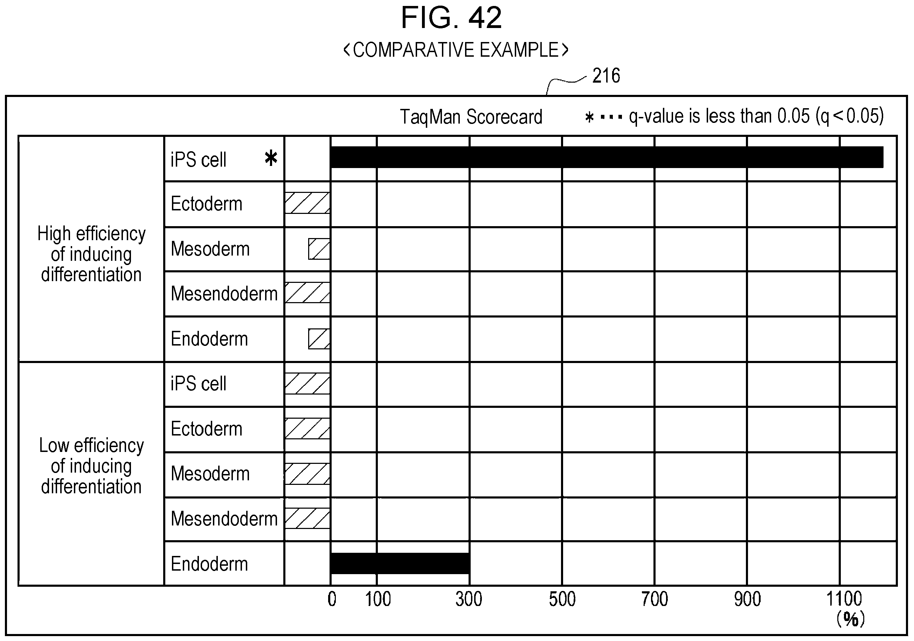

To compare the analysis capabilities, the types of biological samples of TaqMan Scorecard were checked against pieces of annotation information associated with the types of biological samples in C1000 by extracting DEGs, and an odds ratio that indicates to what degree DEGs are concentrated in genes to which each piece of annotation information is added was checked. Fig. 41 illustrates a bar chart 215 of odds ratios based on the set of measurement genes in C1000, and Fig. 42 illustrates a bar chart 216 of odds ratios based on the set of measurement genes of TaqMan Scorecard.

According to the bar chart 215 in Fig. 41, for the set of measurement genes in C1000, concentration in genes related to "mesoderm" and "endoderm" was seen and genes related to "iPS cell" decreased in samples showing a low efficiency of inducing differentiation. For genes related to the types of biological samples in a case of a low efficiency of inducing differentiation, the odds ratios statistically significantly deviated from 100% (the q-values were less than 0.05 (q < 0.05)) except "ectoderm". Accordingly, it was found that the set of the measurement genes in C1000 had a certain analysis capability for samples showing a low efficiency of inducing differentiation. The conceivable reason of such a result is that a sufficiently large number of measurement target genes are distributed in a balanced manner by focusing on each type of biological sample.

According to the bar chart 216 in Fig. 42, for the set of measurement genes of TaqMan Scorecard, concentration in genes related to "iPS cell" was seen in samples showing a high efficiency of inducing differentiation, and concentration in genes related to "endoderm" was seen in samples showing a low efficiency of inducing differentiation. However, only for genes related to "iPS cell" in a case of a high efficiency of inducing differentiation, the odds ratio statistically significantly deviated from 100%. Accordingly, it was found that the set of measurement genes of TaqMan Scorecard had a limited analysis capability for samples showing a low efficiency of inducing differentiation. The conceivable reason of such a result is that unlike in the case of C1000, the number of genes distributed to each type of biological sample is small and an extreme ratio is likely to be produced.

As described above, the technique of the present disclosure enables statistically significant elucidation even when knowledge is not accumulated in advance. That is, a relatively low-cost PCR-based method taking a short test time can be used like RNA-seq, and the technique of the present disclosure can be expected to be widely applicable.

Although the addition number is derived as the evaluation value as is in the above-described embodiments, the technique of the present disclosure is not limited to this. For example, an evaluation value 0 may be derived for an addition number 0, an evaluation value 1 may be derived for addition numbers 1 to 10, and an evaluation value 2 may be derived for addition numbers 11 to 20, that is, a preset evaluation value may be derived in accordance with the addition number.

The form in which measurement target genes are presented to the user is not limited to the form in which the measurement target gene display screen 120 illustrated in Fig. 24 is displayed on the display 49. A form in which the measurement target gene list 78 is printed or a form in which the measurement target gene list 78 is delivered to a terminal owned by the user by, for example, email may be employed. The form in which the pieces of highly influential annotation information 167 are presented to the user in the fourth embodiment described above is not limited to the form in which the highly influential annotation information display screen 180 is displayed on the display 49. A form in which the pieces of highly influential annotation information 167 are printed or a form in which the pieces of highly influential annotation information 167 are delivered to a terminal owned by the user by, for example, email may be employed.

Although the iPS cell 25 has been described as an example of the biological sample that is a study subject in the above-described embodiments, the biological sample is not limited to the iPS cell 25. ES cells, extracts from cells that are being cultured, or a piece of biological tissue may be the biological sample. Although genes have been described as an example of biomarkers, the biomarkers are not limited to genes. Instead of or in addition to genes, gene sequences, gene variants, gene expression, gene modifications, DNA (Deoxyribonucleic acid), epigenome, mRNA (messenger RNA), miRNA (microRNA), protein expressed by cells during culture, metabolites produced from cells during culture, or elements related to the environment for culturing cells, such as the carbon dioxide concentration and pH, may be used as the biomarkers. However, a large number of types of genes exist, and genes are considered to contribute to elucidation of cell behaviors to a larger degree, and therefore, it is preferable to include genes in the biomarkers. As is found from the examples listed above, "biomarker" herein is simply a general term of a substance that indicates various biological feature values.

Various modifications can be made to the hardware configuration of the computer that forms the information processing apparatus 10. The information processing apparatus 10 can be formed of a plurality of computers that are separate hardware devices in order to increase the processing capacity and reliability. For example, two computers may be responsible for the functions of the instruction accepting unit 60, the extraction unit 61, and the acquisition unit 62 and the functions of the deriving unit 63, the selection unit 64, and the display control unit 65 respectively in a distributed manner. In this case, the two computers form the information processing apparatus 10.

As described above, the hardware configuration of the computer that forms the information processing apparatus 10 can be changed as appropriate in accordance with required performance capabilities, such as the processing capacity, safety, and reliability. In addition to the hardware, the application programs including the operation program 55 can be duplicated or stored in a plurality of storage devices in a distributed manner in order to attain safety and reliability.

In the embodiments described above, the hardware configuration of processing units, such as the instruction accepting unit 60, the extraction unit 61, the acquisition unit 62, the deriving unit 63, the selection unit 64, the display control unit 65, and the picking unit 165, that perform various types of processing is implemented as various processors as described below. The various processors include, in addition to the CPU 47, which is a general-purpose processor executing software (the operation program 55) to function as various processing units as described above, a programmable logic device (PLD), such as an FPGA (field-programmable gate array), which is a processor having a circuit configuration that is changeable after manufacture, and a dedicated electric circuit, such as an ASIC (application-specific integrated circuit), which is a processor having a circuit configuration specifically designed to perform specific processing.

One processing unit may be configured as one of the various processors or a combination of two or more processors of the same type or different types (for example, a combination of a plurality of FPGAs and/or a combination of a CPU and an FPGA). Further, a plurality of processing units may be configured as one processor.

As the first example of configuring a plurality of processing units as one processor, a form is possible where one or more CPUs and software are combined to configure one processor, and the processor functions as the plurality of processing units, representative examples of which include computers, such as a client and a server. As the second example thereof, a form is possible where a processor is used in which the functions of the entire system including the plurality of processing units are implemented as one IC (integrated circuit) chip, a representative example of which is a system on chip (SoC). As described above, regarding the hardware configuration, the various processing units are configured by using one or more of the various processors described above.

Further, as the hardware configuration of the various processors, more specifically, an electric circuit (circuitry) in which circuit elements, such as semiconductor elements, are combined can be used.

As the technique of the present disclosure, any of the various embodiments described above and any of the various modifications can be combined as appropriate. In addition to the above-described embodiments, various configurations can be employed without departing from the gist as a matter of course. The technique of the present disclosure embraces not only the program but also a non-transitory storage medium that stores the program.

The content of the description given above and the content of the drawings are detailed explanations of specifics related to the technique of the present disclosure and only show an example of the technique of the present disclosure. For example, explanations of the configurations, functions, operations, and effects described above are explanations of an example of the configurations, functions, operations, and effects of specifics related to the technique of the present disclosure. Therefore, an unwanted part may be deleted from, a new element may be added to, or an element may be replaced in the content of description given above or the content of the drawings without departing from the spirit of the technique of the present disclosure as a matter of course. To avoid complication and facilitate understanding of specifics related to the technique of the present disclosure, explanations of technical common knowledge and so on that do not specifically require explanations for implementing the technique of the present disclosure are omitted from the content of the description given above and the content of drawings.

In this specification, "A and/or B" is synonymous with "at least one of A or B". That is, "A and/or B" may mean only A, may mean only B, or may mean a combination of A and B. In this specification, an idea similar to that of "A and/or B" is applicable to an expression including three or more matters that are joined together by "and/or".

All documents, patent applications, and technical standards described in this specification are incorporated herein by reference to the same extent as in a case where the documents, patent applications, and technical standards are specifically and individually described as being incorporated herein by reference.

## Claims

1. An operation method for an information processing apparatus, the operation method being performed by a processor, the operation method comprising:
an acquisition process for acquiring pieces of annotation information added to each of a plurality of biomarkers related to biological samples;
a deriving process for deriving an evaluation value of each of the plurality of biomarkers on the basis of the pieces of annotation information; and
a selection process for selecting on the basis of the evaluation value, measurement target biomarkers from among the plurality of biomarkers.

2. The operation method for an information processing apparatus according to claim 1, wherein
the processor is configured to
select pieces of annotation information related to a biological-sample's characteristic of interest, and
derive the evaluation value on the basis of only the selected pieces of annotation information.

3. The operation method for an information processing apparatus according to claim 1 or 2, wherein
the processor is configured to
add the pieces of annotation information to the biomarkers with reference to a database in which the pieces of annotation information for the biomarkers are registered.

4. The operation method for an information processing apparatus according to any one of claims 1 to 3, wherein the pieces of annotation information are associated with types of the biological samples.

5. The operation method for an information processing apparatus according to claim 4, wherein
the processor is configured to
accept a plurality of categories defined in accordance with the types of the biological samples and a range of the number of measurement target biomarkers for each of the plurality of categories, the plurality of categories and the range being specified by a user, and
select a number of biomarkers, the number satisfying the range, from among biomarkers prepared for each category of the plurality of categories, and sort the selected biomarkers into the category as the measurement target biomarkers.

6. The operation method for an information processing apparatus according to claim 5, wherein the categories include categories of iPS cell, ectoderm, mesoderm, and endoderm.

7. The operation method for an information processing apparatus according to any one of claims 1 to 6, wherein
the processor is configured to
count an addition number that is the number of pieces of annotation information added to each of the plurality of biomarkers, and
derive the evaluation value on the basis of the addition number.

8. The operation method for an information processing apparatus according to any one of claims 1 to 7, wherein
the processor is configured to
assign a weight to the evaluation value in accordance with information values of the pieces of annotation information.

9. The operation method for an information processing apparatus according to claim 8, wherein
the processor is configured to
determine a piece of annotation information having a relatively high rarity to have a high information value and increase a weight of the piece of annotation information.

10. The operation method for an information processing apparatus according to claim 8 or 9, wherein
the processor is configured to
assign the weight to the evaluation value on the basis of orthogonality of the pieces of annotation information.

11. The operation method for an information processing apparatus according to any one of claims 1 to 10, wherein
the processor is configured to
increase a weight of an evaluation value of a biomarker having a strength indicator that is within a preset threshold range.

12. The operation method for an information processing apparatus according to any one of claims 1 to 11, wherein
the processor is configured to
accept previously known markers specified by a user, the previously known markers being biomarkers already known to influence a characteristic of the biological samples, and
increase weights of evaluation values of the previously known markers.

13. The operation method for an information processing apparatus according to any one of claims 1 to 12, wherein
the processor is configured to
select as the measurement target biomarkers, more than 100 and less than or equal to 1000 biomarkers.

14. The operation method for an information processing apparatus according to any one of claims 1 to 13, wherein the biomarkers include genes.

15. The operation method for an information processing apparatus according to claim 14, wherein the genes include differentially expressed genes having significantly different expression levels.

16. The operation method for an information processing apparatus according to any one of claims 1 to 15, wherein each of the pieces of annotation information includes a term defined by Gene Ontology.

17. The operation method for an information processing apparatus according to any one of claims 1 to 16, wherein
the processor is configured to
acquire measurement results regarding the measurement target biomarkers,
pick, on the basis of the measurement results, pieces of annotation information that influence a characteristic of the biological samples to a relatively large degree from among pieces of annotation information added to the measurement target biomarkers, with a statistical method, and
present the picked pieces of annotation information to a user.

18. An information processing apparatus comprising
at least one processor,
the at least one processor being configured to
acquire pieces of annotation information added to each of a plurality of biomarkers related to biological samples,
derive an evaluation value of each of the plurality of biomarkers on the basis of the pieces of annotation information, and
select on the basis of the evaluation value, measurement target biomarkers from among the plurality of biomarkers.

19. An operation program for an information processing apparatus for causing a processor to perform
an acquisition process for acquiring pieces of annotation information added to each of a plurality of biomarkers related to biological samples;
a deriving process for deriving an evaluation value of each of the plurality of biomarkers on the basis of the pieces of annotation information; and
a selection process for selecting on the basis of the evaluation value, measurement target biomarkers from among the plurality of biomarkers.
